# FASCICULE DE BREVET EUROPEEN

(11) **EP 1 457 199 B1**
(45) Date de publication et mention de la délivrance du brevet: **14.05.2008**
(21) Numéro de dépôt: 04290611.5
(22) Date de dépôt: 05.03.2004
(51) Int. Cl.: A61K 8/49, A61Q 5/10, C07D 413/04, C07D 213/74, C07D 213/61, C07D 231/38, C07D 239/46, C07D 401/04, C07D 471/04

(54) **Nouveaux coupleurs du type 2,3,5-triaminopyridine et utilisation de ces coupleurs pour la teinture des fibres kératiniques**
Kupplersubstanzen mit 2,3,5-Triaminopyridin-Struktur und deren Verwendung zum Färben von Keratinfasern
Coupling agents having a 2,3,5-triaminopyridine structure and their use for dyeing keratinic fibres

(30) Priorité: 13.03.2003 FR 0303114
(43) Date de publication de la demande: 15.09.2004
(73) Titulaire: L'ORÉAL, 75008 Paris (FR)
(72) Inventeur: Fadli, Aziz, 77500 Chelles (FR); Vidal, Laurent, 75013 Paris (FR)
(74) Mandataire: Fevrier, Murielle Françoise E.

(56) Documents cités:
- DE-A- 1 949 750
- FR-A- 1 397 551
- US-A- 4 734 418
- CRAMPTON, MICHAEL R. ET AL: "Kinetic and equilibrium studies of.sigma.-adduct formation and nucleophilic substitution in the reactions of 2-phenoxy-3,5- dinitropyridine and 2-ethoxy-3,5-dinitropyridine with aliphatic amines in dipolar aprotic solvents" ORGANIC & BIOMOLECULAR CHEMISTRY (2003), vol. 1, no. 6, 20 février 2003 (2003-02-20), pages 1004-1011, XP001156850
- ELHEGAZY, FATMA EL ZAHRAA M.: "Kinetics of nucleophilic aromatic substitution of morpholine with halo nitro compounds. Comparison of activation between aza and nitro groups" ALEXANDRIA JOURNAL OF PHARMACEUTICAL SCIENCES (1996), vol. 10, no. 2, juin 1996 (1996-06), pages 113-116, XP009023271
- DATABASE CA [en ligne] CHEMICAL ABSTRACTS SERVICE, COLUMBUS, OHIO, US; SIGNOR, ANGELO ET AL: "Structure of proteins. VII. Preparation of nitropyridylamino acids" retrieved from STN Database accession no. 59:62817 XP002265349 & GAZZETTA CHIMICA ITALIANA (1963), 93(1-2), 73-80,

## Description

L'invention a pour objet une composition tinctoriale utile pour la teinture des fibres kératiniques contenant au moins une base d'oxydation et au moins un coupleur du type 2,3,5-triaminopyridine dont le radical amino en position 2 forme un radical hétérocyclique, l'utilisation de cette composition pour la teinture des fibres kératiniques ainsi que le procédé de teinture mettant en oeuvre cette composition. L'invention a aussi pour objet de nouveaux composés du type 2,3,5-triaminopyridines utiles comme coupleurs.

Il est connu de teindre les fibres kératiniques et en particulier les cheveux humains avec des compositions tinctoriales contenant des précurseurs de colorant d'oxydation, appelés généralement bases d'oxydation, tels que des ortho ou paraphénylènediamines, des ortho ou para-aminophénols et des composés hétérocycliques. Ces bases d'oxydation sont des composés incolores ou faiblement colorés qui, associés à des produits oxydants, peuvent donner naissance par un processus de condensation oxydative à des composés colorés.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques tels que des composés indoliques.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs tels que la lumière, les intempéries, le lavage, les ondulations permanentes, la transpiration et les frottements.

Les colorants doivent également permettre de couvrir les cheveux blancs, et être enfin les moins sélectifs possibles, c'est-à-dire permettre d'obtenir des écarts de coloration les plus faibles possibles tout au long d'une même fibre kératinique, qui est en général différemment sensibilisée (i.e. abîmée) entre sa pointe et sa racine.

Le document FR 1397551 décrit des compositions tinctoriales contenant des précurseurs de colorant d'oxydation du type dérivé pyridinique tri-substitués, chacun des substituants pouvant être un radical hydroxy, alcoxy, amino, ou NR₁R₂ avec R₁R₂ représentant un H, alkyle, aryle. La coloration est obtenue soit par oxydation à l'air soit par un milieu oxydant contenant de l'eau oxygénée à pH basique. En raison de la forte oxydabilité de ces précurseurs pyridiniques, les teintures obtenues sur cheveux ont tendance à évoluer dans le temps en changeant de couleur, ce qui s'avère particulièrement inesthétique.

Toutes ces compositions ne permettent cependant pas d'obtenir des colorations intenses dans des nuances variées qui sont uniformes entre la racine et la pointe des cheveux, peu sélectives et particulièrement résistantes, et présentent une bonne chromaticité.

Le but de la présente invention est de proposer des compositions tinctoriales pour la coloration des fibres kératiniques qui ne présentent pas les inconvénients des compositions de la technique antérieure. En particulier, le but de l'invention est de proposer des compositions qui permettent d'obtenir des teintures puissantes, uniformes entre la pointe et la racine, résistante aux agents extérieurs, tout en étant capables de donner des nuances variées en particulier dans des nuances fondamentales telles que des nuances chatain, gris ou noir.

Ce but est atteint avec la présente invention qui a pour objet une composition tinctoriale comprenant, dans un milieu de teinture approprié :
- au moins une base d'oxydation, et
- au moins un coupleur 2,3,5-triaminopyridine de formule (I) ou l'un de ses sels d'addition correspondants : 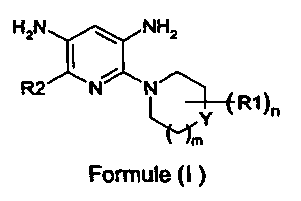
dans laquelle :
- R₁ représente :
   - un atome d'halogène (tel que fluor, chlore, brome) ;
   - un alkyle en C₁-C₄ éventuellement substitué par un ou plusieurs radicaux choisis parmi les radicaux hydroxy, carboxy, alcoxycarbonyle en C₁-C₄, carboxamido, alkyl(C₁-C₄)sulfonyle, alcoxy en C₁-C₄, alkyl(C₁-C₄)sulfonamido, NR₃R₄;
   - un radical carboxy ;
   - un radical alcoxycarbonyle en C₁-C₄ ;
   - un radical carboxamido ; (NH₂CO-)
   - un radical alkyl en C₁-C₄ carboxamido ; (alkyl-NHCO- ou (alkyl)₂NCO-)
   - un radical sulfinique (HSO₂-)
   - un radical alkyl(C₁-C₄)sulfonyle ; (-SO₂-alkyl)
   - un radical alkyl(C₁-C₄)sulfonamido ; (alkylSO₂NH-)
   - un radical hydroxy ;
   - un radical alcoxy en C₁-C₄ ;
   - un radical hydroxyalcoxy en C₁-C₄ ;
   - un radical amino ou mono- ou di- amino alcoxy;
   - un radical thioéther en C₁-C₄ ;
   - un radical alkyl(C₁-C₄)sulfoxyde ; (alkylSO-) ;
   - un radical sulfonique (-SO3H) ;
   - un radical NR₅R₆ ;
- R₃, R₄, R₅ et R₆ représentent, identiques ou différents, un atome d'hydrogène ; un radical alkyl(C₁-C₄)sulfonyle ; un radical alkyle(C₁-C₄)carbonyle dans lequel le radical alkyle peut être substitué par un ou plusieurs hydroxy ; un radical arylcarbonyle, le radical aryle pouvant être substitué par un radical choisi parmi hydroxy, alcoxy en C₁-C₄, amino ou (di)alkyl(C₁-C₄)amino ; un radical carboxamido ; un radical alkyle en C₁-C₄ éventuellement substitué par un ou plusieurs radicaux choisis parmi les radicaux hydroxy, alcoxy en C₁-C₄, alkyl(C₁-C₄)sulfonyle, alkyl(C₁-C₄)sulfonamido, carboxy, carboxamido, alkyl(C₁-C₄)sulfoxyde, amino ,(di)(C₁-C₄)alkylamino ou (poly)hydroxyalkylamino en C₂-C₄ ;
- R₂ représente un atome d'hydrogène ; un radical alcoxy en C₁-C₄ éventuellement substitué par un ou plusieurs radicaux hydroxy, alcoxy en C₁-C₂,
- n est un nombre entier compris entre 0 et 7, inclus
- m est 0, 1 ou 2,
- Y représente un atome d'oxygène, un radical C(R₈)₂ ou un radical NR₇ où R₇ a la même signification que R₃ et R₈, identiques ou différents, représentent l'hydrogène ou ont la même signification que R₁.

La présente invention a aussi pour objet un procédé de teinture des fibres kératiniques ainsi qu'un dispositif de teinture à partir de la composition de l'invention.

Enfin, l'invention a pour objet les composés de formule (I) ainsi que les composés nitro intermédiaires dans la synthèse des composés de formule (I).

Dans le cadre de la présente invention, on entend par alkyle, des radicaux linéaires ou ramifiés par exemple méthyle, éthyle, n-propyle, iso-propyle, butyle, etc. Un radical alcoxy est un radical alkyle-O, le radical alkyle ayant la définition donnée ci dessus.

Dans la formule ci dessus, R₁ représente de préférence un radical alcoxy en C1-C4 éventuellement substitué par un ou plusieurs radicaux hydroxy, alcoxy en C1-C2, amino ou (di)alkylamino; un radical hydroxy; un radical amino; un radical (di)alkylamino ; un radical alkyle en C1-C2 éventuellement substitué par un hydroxy, amino.

R2 représente selon un mode de réalisation préféré un atome d'hydrogène ou un radical alcoxy.

Dans la formule (I) ci dessus, n représente de préférence 0 ou 1.

R₃, R₄, R₅, R₆ et R₇, identiques ou différents, peuvent représenter un atome d'hydrogène, un radical carboxamido, un radical alkyle en C₁-C₄ éventuellement substitué par un ou plusieurs radicaux choisis parmi les radicaux hydroxy, alcoxy en C₁-C₄, carboxamido, amino, (di)(C₁-C₄)alkylamino, (poly)hydroxyalkylamino en C₂-C₄.

De préférence, R₃, R₄, R₅, R₆ et R₇ représentent, identiques ou différents, un atome d'hydrogène, un radical méthyle, un éthyle, 2-carboxyéthyle, 2-hydroxyéthyle, 3-hydroxypropyle, 2,3-dihydroxypropyle, 2-hydroxy-3-aminopropyle, 3-hydroxy-2-aminopropyle. Selon un mode encore préféré, R₃, R₄, R₅, R₆ et R₇ représentent, identiques ou différents un atome d'hydrogène, un radical méthyle, un radical 2-hydroxyéthyle, un radical 2,3-dihydroxypropyle.

De préférence, au moins l'un des radicaux R₈ représente l'hdyrogène.

Dans la formule (I), l'azote en position 2 du cycle, Y et m peuvent former un radical hétérocyclique choisi parmi les pyrrolidines, les pipéridines, les homopipéridines, les pipérazines, les homopipérazines, les diazépanes (ou 1,4-diazacycloheptane).

Selon un mode de réalisation particulier, l'hétérocycle est choisi parmi la pyrrolidine, la 2,5-diméthylpyrrolidine, la 2-méthylpyrrolidine la proline, la 3-hydroxyproline, la 4-hydroxyproline, la 2,4-dicarboxypyrrolidine, la 2-hydroxyméthylpyrrolidine, la 3-hydroxy-2-hydroxyméthylpyrrolidine, la 2,5-di(hydroxyméthyl)pyrrolidine, la 2-carboxamidopyrrolidine, la 3-hydroxy-2-carboxamidopyrrolidine, la 2-(diméthylcarboxamido)pyrrolidine, la 2-(diméthylcarboxamido)-3-hydroxy-pyrrolidine, la 3,4-dihydroxy-2-hydroxyméthyl pyrrolidine, la 3-hydroxypyrrolidine, la 3,4-dihydroxypyrrolidine, la 3-aminopyrrolidine, la 3-méthylaminopyrrolidine, la 3-diméthylaminopyrrolidine, la 4-amino-3-hydroxy pyrrolidine, la 4-méthylamino-3-hydroxypyrrolidine, la 3-hydroxy-4-(2-hydroxyéthyl)amino-pyrrolidine, la pipéridine, la 2,6-diméthylpipéridine, la 2-carboxypipéridine, la 2-carboxamidopipéridine, la 2-(diméthylcarboxamido)pipéridine, la 2-hydroxyméthylpipéridine, la 3-hydroxy-2-hydroxyméthylpipéridine, 3-hydroxypipéridine, la 4-hydroxypipéridine, la 3-hydroxyméthylpipéridine, l'homopipéridine, la 2-carboxyhomopipéridine, la 2-carboxamidohomopipéridine, la pipérazine, la 4-méthylpipérazine le diazépane, N-méthyl homopipérazine, la N β-hydroxyéthylhomopipérazine et leurs sels d'addition.

De préférence, l'hétérocycle peut être choisi parmi la pyrrolidine, la 2-méthylpyrrolidine, la 3-hydroxypyrrolidine, la 3-aminopyrrolidine, la 3-(méthylsulfonylamino)-pyrrolidine, la proline, la 3-hydroxyproline, la pipéridine, l'hydroxypipéridine, l'homopipéridine, la 4-méthylpipérazine, le diazépane, N-méthyl homopipérazine, la N β-hydroxyéthylhomopipérazine et leurs sels d'addition.

Selon un mode de réalisation particulièrement préféré, l'hétérocycle est choisi parmi la pyrrolidine, la 3-hydroxypyrrolidine, la 3-aminopyrrolidine, la 3-(méthylsulfonylamino)-pyrrolidine, la proline, la 3-hydroxyproline .

Selon un mode de réalisation particulier, dans la formule (I), R1 représente un radical alkyle, amino, hydroxyalkyle, hydroxy, R2 est l'hydrogène, m est 0 ou 1, et n est compris entre 0 et 2.

Les composés de formule (I) utiles dans la présente invention sont par exemple les composés
N-(3,5-diaminopyridin-2-yl)pyrrolidine
N-(3,5-diaminopyridin-2-yl)-2-méthylpyrrolidine
N-(3,5-diaminopyridin-2-yl)-2,5-diméthylpyrrolidine
N-(3,5-diaminopyridin-2-yl)-2-hydroxyméthylpyrrolidine
N-(3,5-diaminopyridin-2-yl)proline
N-(3,5-diaminopyridin-2-yl)-3-hydroxypyrrolidine
N-(3,5-diaminopyridin-2-yl)-2-hydroxyméthyl-3-hydroxypyrrolidine
N-(3,5-diaminopyridin-2-yl)-2-carboxamidopyrrolidine
N-(3,5-diaminopyridin-2-yl)-2-diméthylcarboxamidopyrrolidine
N-(3,5-diaminopyridin-2-yl)-3,4-dihydroxypyrrolidine
N-(3,5-diaminopyridin-2-yl)-3-aminopyrrolidine
N-(3,5-diaminopyridin-2-yl)-3-diméthylaminopyrrolidine
N-(3,5-diaminopyridin-2-yl)pipéridine
N-(3,5-diaminopyridin-2-yl)-2,5-diméthylpipéridine
N-(3,5-diaminopyridin-2-yl)-2-hydroxyméthylpipéridine
N-(3,5-diaminopyridin-2-yl)-2-carboxypipéridine
N-(3,5-diaminopyridin-2-yl)-3-hydroxypipéridine
N-(3,5-diaminopyridin-2-yl)-2-carboxamidopipéridine
N-(3,5-diaminopyridin-2-yl)-2-diméthylcarboxamidopipéridine
N-(3,5-diaminopyridin-2-yl)-4-hydroxypipéridine
N-(3,5-diaminopyridin-2-yl)homopipéridine
N-(3,5-diaminopyridin-2-yl)-2-carboxyhomopipéridine
N-(3,5-diaminopyridin-2-yl)-4-méthylpipérazine
N-(3,5-diaminopyridin-2-yl)-homopipérazine
N-(3,5-diaminopyridin-2-yl)-N'-méthylhomopipérazine
N-(3,5-diaminopyridin-2-yl)-6-méthoxy-pyrrolidine
N-(3,5-diaminopyridin-2-yl)-6-méthoxy-2,5-diméthylpyrrolidine
N-(3,5-diaminopyridin-2-yl)-6-méthoxy-2-hydroxyméthylpyrrolidine
N-(3,5-diaminopyridin-2-yl)-6-méthoxy-proline
N-(3,5-diaminopyridin-2-yl)-6-méthoxy-3-hydroxypyrrolidine
N-(3,5-diaminopyridin-2-yl)-6-méthoxy-2-hydroxyméthyl-3-hydroxypyrrolidine
N-(3,5-diaminopyridin-2-y1)-6-méthoxy-2-carboxamidopyrrolidine
N-(3,5-diaminopyridin-2-yl)-6-méthoxy-2-diméthylcarboxamidopyrrolidine
N-(3,5-diaminopyridin-2-yl)-6-méthoxy-3,4-dihydroxypyrrolidine
N-(3,5-diaminopyridin-2-yl)-6-méthoxy-3-aminopyrrolidine
N-(3,5-diaminopyridin-2-yl)-6-méthoxy-3-diméthylaminopyrrolidine
N-(3,5-diaminopyridin-2-yl)-6-méthoxy-pipéridine
N-(3,5-diaminopyridin-2-yl)-méthoxy-2,5-diméthylipéridine
N-(3,5-diaminopyridin-2-yl)-6-méthoxy-2-hydroxyméthylpipéridine
N-(3,5-diaminopyridin-2-yl)-6-méthoxy-2-carboxypipéridine
N-(3,5-diaminopyridin-2-yl)-6-méthoxy-3-hydroxypipéridine
N-(3,5-diaminopyridin-2-yl)-6-méthoxy-2-carboxamidopipéridine
N-(3,5-diaminopyridin-2-yl)-6-méthoxy-2-diméthylcarboxamidopipéridine
N-(3,5-diaminopyridin-2-yl)-6-méthoxy-4-hydroxypipéridine
N-(3,5-diaminopyridin-2-yl)-6-méthoxy-homopipéridine
N-(3,5-diaminopyridin-2-yl)-6-méthoxy-2-carboxyhomopipéridine
N-(3,5-diaminopyridin-2-yl)-6-méthoxy-homopipérazine
N-(3,5-diaminopyridin-2-yl)-6-méthoxy-N'-méthylhomopipérazine et leurs sels d'addition.

Les composés de formule (I) plus particulièrement préférés sont choisis parmi :
N-(3,5-diaminopyridin-2-yl)pyrrolidine
N-(3,5-diaminopyridin-2-yl)-2-méthylpyrrolidine
N-(3,5-diaminopyridin-2-yl)-2,5-diméthylpyrrolidine
N-(3,5-diaminopyridin-2-yl)-2-hydroxyméthylpyrrolidine
N-(3,5-diaminopyridin-2-yl)proline
N-(3,5-diaminopyridin-2-yl)-3-hydroxypyrrolidine
N-(3,5-diaminopyridin-2-yl)-2-carboxamidopyrrolidine
N-(3,5-diaminopyridin-2-yl)-2-diméthylcarboxamidopyrrolidine
N-(3,5-diaminopyridin-2-yl)-3,4-dihydroxypyrrolidine
N-(3,5-diaminopyridin-2-yl)-3-aminopyrrolidine
N-(3,5-diaminopyridin-2-yl)-3-diméthylaminopyrrolidine
N-(3,5-diaminopyridin-2-yl)pipéridine
N-(3,5-diaminopyridin-2-yl)-4-méthylpipérazine
ainsi que leurs sels d'addition.

La composition de teinture par oxydation de la présente invention comprend une ou plusieurs bases d'oxydation classiquement utilisées en teinture d'oxydation. A titre d'exemple, ces bases d'oxydation sont choisies parmi les para-phénylènediamines autres que celles décrites précédemment, les bis-phénylalkylènediamines, les para-aminophénols, les bis-para-aminophénols, les ortho-aminophénols, les bases hétérocycliques et leurs sels d'addition.

Parmi les para-phénylènediamines, on peut citer à titre d'exemple, la para-phénylènediamine, la para-toluènediamine, la 2-chloro para-phénylènediamine, la 2,3-diméthyl para-phénylènediamine, la 2,6-diméthyl para-phénylènediamine, la 2,6-diéthyl para-phénylènediamine, la 2,5-diméthyl para-phénylènediamine, la N,N-diméthyl para-phénylènediamine, la N,N-diéthyl para-phénylènediamine, la N,N-dipropyl para-phénylènediamine, la 4-amino N,N-diéthyl 3-méthyl aniline, la N,N-bis-(β-hydroxyéthyl) para-phénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl para-phénylènediamine, la 2-fluoro para-phénylènediamine, la 2-isopropyl para-phénylènediamine, la N-(β-hydroxypropyl) para-phénylènediamine, la 2-hydroxyméthyl para-phénylènediamine, la N,N-diméthyl 3-méthyl para-phénylènediamine, la N,N-(éthyl, β-hydroxyéthyl) para-phénylènediamine, la N-(β,γ-dihydroxypropyl) para-phénylènediamine, la N-(4'-aminophényl) para-phénylènediamine, la N-phényl para-phénylènediamine, la 2-β-hydroxyéthyloxy para-phénylènediamine, la 2-β-acétylaminoéthyloxy para-phénylènediamine, la N-(β-méthoxyéthyl) para-phénylènediamine, la 4-aminophénylpyrrolidine, la 2-thiényl para-phénylènediamine, le 2-β hydroxyéthylamino 5-amino toluène, la 3-hydroxy 1-(4'-aminophényl)pyrrolidine et leurs sels d'addition avec un acide.

Parmi les para-phénylènediamines citées ci-dessus, la para-phénylènediamine, la para-toluènediamine, la 2-isopropyl para-phénylènediamine, la 2-β-hydroxyéthyl para-phénylènediamine, la 2-β-hydroxyéthyloxy para-phénylènediamine, la 2,6-diméthyl para-phénylènediamine, la 2,6-diéthyl para-phénylènediamine, la 2,3-diméthyl para-phénylènediamine, la N,N-bis-(β-hydroxyéthyl) para-phénylènediamine, la 2-chloro para-phénylènediamine, la 2-β-acétylaminoéthyloxy para-phénylènediamine, et leurs sels d'addition avec un acide sont particulièrement préférées.

Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition avec un acide.

Parmi les para-aminophénols, on peut citer à titre d'exemple, le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques, on peut citer à titre d'exemple, les dérivés pyridiniques, les dérivés pyrimidiniques et les dérivés pyrazoliques.

Parmi les dérivés pyridiniques, on peut citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition avec un acide.

D'autres bases d'oxydation pyridiniques utiles dans la présente invention sont les bases d'oxydation 3-amino pyrazolo-[1,5-a]-pyridines ou leurs sels d'addition décrits par exemple dans la demande de brevet FR 2801308. A titre d'exemple, on peut citer la pyrazolo[1,5-a]pyridin-3-ylamine ; la 2-acétylamino pyrazolo-[1,5-a] pyridin-3-ylamine ; la 2-morpholin-4-yl-pyrazolo[1,5-a]pyridin-3-ylamine ; l'acide 3-amino-pyrazolo[1,5-a]pyridin-2-carboxylique ; la 2-méthoxy-pyrazolo[1,5-a]pyridine-3-ylamino ; le (3-amino-pyrazolo[1,5-a]pyridine-7-yl)-méthanol ; le 2-(3-amino-pyrazolo[1,5-a]pyridine-5-yl)-éthanol ; le 2-(3-amino-pyrazolo[1,5-a]pyridine-7-yl)-éthanol ; le (3-amino-pyrazolo[1,5-a]pyridine-2-yl)-méthanol ; la 3,6-diamino-pyrazolo[1,5-a]pyridine ; la 3,4-diamino-pyrazolo[1,5-a]pyridine ; la pyrazolo[1,5-a]pyridine-3,7-diamine ; la 7-morpholin-4-yl-pyrazolo[1,5-a]pyridin-3-ylamine ; la pyrazolo[1,5-a]pyridine-3,5-diamine ; la 5-morpholin-4-yl-pyrazolo[1,5-a]pyridin-3-ylamine ; le 2-[(3-amino-pyrazolo[1,5-a]pyridin-5-yl)-(2-hydroxyéthyl)-amino]-éthanol ; le 2-[(3-amino-pyrazolo[1,5-a]pyridin-7-yl)-(2-hydroxyéthyl)-amino]-éthanol ; la 3-amino-pyrazolo[1,5-a]pyridine-5-ol ; 3-amino-pyrazolo[1,5-a]pyridine-4-ol ; la 3-amino-pyrazolo[1,5-a]pyridine-6-ol ; la 3-amino-pyrazolo[1,5-a]pyridine-7-ol ; ainsi que leurs d'addition avec un acide ou avec une base.

Parmi les dérivés pyrimidiniques, on peut citer les composés décrits par exemple dans les brevets DE 2359399 ; JP 88-169571 ; JP 05-63124 ; EP 0770375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, et les dérivés pyrazolo-pyrimidiniques tels ceux mentionnés dans la demande de brevet FR-A-2750048 et parmi lesquels on peut citer la pyrazolo-[1,5-a]-pyrimidine-3,7-diamine ; la 2,5-diméthyl pyrazolo-[1,5-a]-pyrimidine-3,7-diamine ; la pyrazolo-[1,5-a]-pyrimidine-3,5-diamine ; la 2,7-diméthyl pyrazolo-[1,5-a]-pyrimidine-3,5-diamine; le 3-amino pyrazolo-[1,5-a]-pyrimidin-7-ol ; le 3-amino pyrazolo-[1,5-a]-pyrimidin-5-ol ; le 2-(3-amino pyrazolo-[1,5-a]-pyrimidin-7-ylamino)-éthanol, le 2-(7-amino pyrazolo-[1,5-a]-pyrimidin-3-ylamino)-éthanol, le 2-[(3-amino-pyrazolo[1,5-a]pyrimidin-7-yl)-(2-hydroxy-éthyl)-amino]-éthanol, le 2-[(7-amino-pyrazolo[1,5-a]pyrimidin-3-yl)-(2-hydroxy-éthyl)-amino]-éthanol, la 5,6-diméthyl pyrazolo-[1,5-a]-pyrimidine-3,7-diamine, la 2,6-diméthyl pyrazolo-[1,5-a]-pyrimidine-3,7-diamine, la 2, 5, N 7, N 7-tetraméthyl pyrazolo-[1,5-a]-pyrimidine-3,7-diamine, la 3-amino-5-méthyl-7-imidazolylpropylamino pyrazolo-[1,5-a]-pyrimidine et leurs sels d'addition avec un acide et leurs formes tautomères, lorsqu'il existe un équilibre tautomérique.

Parmi les dérivés pyrazoliques, on peut citer les composés décrits dans les brevets DE 3843892, DE 4133957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

La ou les bases d'oxydation présentes dans la composition de l'invention sont en général présentent chacune en quantité comprise entre 0,001 à 10 % en poids environ du poids total de la composition tinctoriale, de préférence entre 0,005 et 6 %.

La composition selon l'invention peut contenir un ou plusieurs coupleurs additionnels conventionnels dans le domaine de la coloration autres que les coupleurs de formule (I). Parmi ces coupleurs additionnels, on peut notamment citer les métaphénylènediamines, les méta-aminophénols, les métadiphénols, les coupleurs naphtaléniques, les coupleurs hétérocycliques et leur sels d'addition.

A titre d'exemple, on peut citer le 2-méthyl 5-aminophénol, le 5-N-(β-hydroxyéthyl)amino 2-méthyl phénol, le 6-chloro-2-méthyl-5-aminophénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6- hydroxy benzomorpholine la 3,5-diamino-2,6-diméthoxypyridine, le 1-N-(β-hydroxyéthyl)amino-3,4-méthylène dioxybenzène, le 2,6-bis-(β-hydroxyéthylamino)toluène et leurs sels d'addition avec un acide.

Dans la composition de la présente invention, le ou les coupleurs sont chacun généralement présents en quantité comprise entre 0,001 et 10 % en poids environ du poids total de la composition tinctoriale, de préférence entre 0,005 et 6 %.

D'une manière générale, les sels d'addition des bases d'oxydation et des coupleurs utilisables dans le cadre de l'invention sont notamment choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates et les sels d'addition avec une base telles que la soude, la potasse, l'ammoniaque, les amines ou les alcanolamines.

La composition tinctoriale conforme à l'invention peut en outre contenir un ou plusieurs colorants directs pouvant notamment être choisis parmi les colorants nitrés de la série benzénique, les colorants directs azoïques, les colorants directs méthiniques. Ces colorants directs peuvent être de nature non ionique, anionique ou cationique.

La composition tinctoriale de la présente invention est particulièrement utile pour la teinture des fibres kératiniques, en particulier les fibres kératiniques humaines. Dans ce cas, le milieu et un milieu cosmétique approprié à la teinture de ces fibres.

Cee milieu approprié pour la teinture appelé aussi support de teinture est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Les solvants sont, de préférence, présents dans des proportions de préférence comprises entre 1 et 40 % en poids environ par rapport au poids total de la composition tinctoriale, et encore plus préférentiellement entre 5 et 30 % en poids environ.

La composition tinctoriale conforme à l'invention peut également renfermer divers adjuvants utilisés classiquement dans les compositions pour la teinture des cheveux, tels que des agents tensio-actifs anioniques, cationiques, non-ioniques, amphotères, zwitterioniques ou leurs mélanges, des polymères anioniques, cationiques, non-ioniques, amphotères, zwitterioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Les adjuvants ci dessus sont en général présents en quantité comprise pour chacun d'eux entre 0,01 et 20 % en poids par rapport au poids de la composition.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

Le pH de la composition tinctoriale conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (III) suivante : 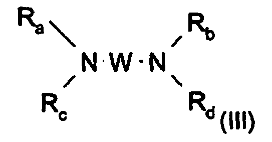 dans laquelle W est un reste propylène éventuellement substitué par un groupement hydroxyle ou un radical alkyle en C₁-C₄ ; Rₐ, R_{b}, R_{c} et R_{d}, identiques ou différents, représentent un atome d'hydrogène, un radical alkyle en C₁-C₄ ou hydroxyalkyle en C₁-C₄.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

Le procédé de la présente invention est un procédé dans lequel on applique sur les fibres la composition selon la présente invention telle que définie précédemment, en présence d'un agent oxydant pendant un temps suffisant pour développer la coloration désirée. La couleur peut être révélée à pH acide, neutre ou alcalin et l'agent oxydant peut être ajouté à la composition de l'invention juste au moment de l'emploi ou il peut être mis en oeuvre à partir d'une composition oxydante le contenant, appliquée simultanément ou séquentiellement à la composition de l'invention.

Selon un mode de réalisation particulier, la composition selon la présente invention est mélangée, de préférence au moment de l'emploi, à une composition contenant, dans un milieu approprié pour la teinture, au moins un agent oxydant, cet agent oxydant étant présent en une quantité suffisante pour développer une coloration. Le mélange obtenu est ensuite appliqué sur les fibres kératiniques. Après un temps de pose de 3 à 50 minutes environ, de préférence 5 à 30 minutes environ, les fibres kératiniques sont rincées, lavées au shampooing, rincées à nouveau puis séchées.

Les agents oxydants classiquement utilisés pour la teinture d'oxydation des fibres kératiniques sont par exemple le peroxyde d'hydrogène, le peroxyde d'urée, les bromates de métaux alcalins, les persels tels que les perborates et persulfates, les peracides et les enzymes oxydases parmi lesquelles on peut citer les peroxydases, les oxydo-réductases à 2 électrons telles que les uricases et les oxygénases à 4 électrons comme les laccases. Le peroxyde d'hydrogène est particulièrement préféré.

La composition oxydante peut également renfermer divers adjuvants utilisés classiquement dans les compositions pour la teinture des cheveux et tels que définis précédemment.

Le pH de la composition oxydante renfermant l'agent oxydant est tel qu'après mélange avec la composition tinctoriale, le pH de la composition résultante appliquée sur les fibres kératiniques varie de préférence entre 3 et 12 environ, et préférentiellement entre 5 et 11. De préférence, le pH est compris entre 6 et 8. Il peut être ajusté à la valeur désirée au moyen d'agents acidifiants ou alcalinisants habituellement utilisés en teinture des fibres kératiniques et tels que définis précédemment.

La composition prête à l'emploi qui est finalement appliquée sur les fibres kératiniques peut se présenter sous des formes diverses, telles que sous forme de liquides, de crèmes, de gels ou sous toute autre forme appropriée pour réaliser une teinture des fibres kératiniques, et notamment des cheveux humains.

La composition de l'invention peut se présenter sous forme de kit. Un tel kit comprend d'une part une composition telle que définie précédemment et d'autre part une composition oxydante.

L'invention a aussi pour objet un dispositif à plusieurs compartiments dans lequel un premier compartiment renferme la composition tinctoriale de la présente invention définie ci-dessus et un deuxième compartiment renferme un agent oxydant. Ce dispositif peut être équipé d'un moyen permettant de délivrer sur les cheveux le mélange souhaité, tel que les dispositifs décrits dans le brevet FR-2 586 913 au nom de la demanderesse.

A partir de ce dispositif, il est possible de teindre les fibres kératiniques à partir d'un procédé qui comprend le mélange d'une composition tinctoriale comprenant de l'invention avec un agent oxydant, et l'application du mélange obtenu sur les fibres kératiniques pendant un temps suffisant pour développer la coloration désirée.

La présente invention a enfin pour objet les composés 2,3,5 triaminopyridine de formule (I) ainsi que leurs sels d'addition correspondants tels que définis précédemment.

Ces composés peuvent être synthétisés selon le schéma de synthèse suivant : 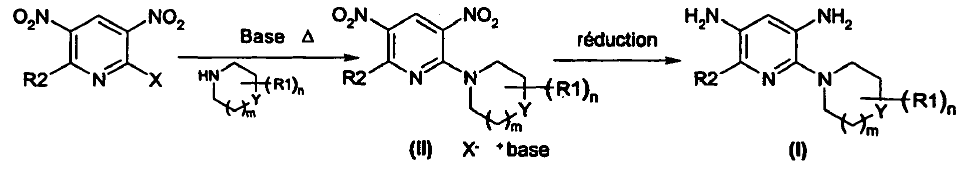 X représentant un atome d'halogène tel qu'un chlore ou brome, ou un radical alcoxy en C₁-C₂ et R2, R1, n et m étant tels que définis précédemment.

Les composés de formule (II) peuvent être obtenus d'une façon générale en solubilisant sous agitation une 2-halogéno-3,5-dinitropyridine ou 2-alcoxy-3,5-dinitropyridine tel que la 2-chloro-3,5-dinitropyridine ou la 2-méthoxy-3,4-dinitropyridine dans un solvant protique ou aprotique de point d'ébullition compris entre 60°C et 180°C tel que par exemple le dioxane, le DMF, le THF, un alcool inférieur, l'eau et en présence d'une base organique ou inorganique pouvant former un sel avec l'ion libéré. L'amine cyclique est ensuite introduite au goutte à goutte. La température du milieu réactionnel est en général comprise entre 25°C et 100°C. Après disparition des réactifs, le milieu réactionnel est refroidi à température ambiante et versé sur un mélange de glace et d'eau. Le précipité ainsi formé est essoré sur fritté, lavé à l'eau puis il est séché sous vide jusqu'à poids constant.

Les composés de formule (I) peuvent ensuite être obtenus en réduisant les précurseurs nitrés de formule (II) soit par hydrogénation catalytique, soit par transfert d'hydrogène, soit par un métal tel que le zinc, l'étain ou le fer, soit par un hydrure tel que le borohydrure de sodium ou l'aluminohydrure de lithium. La réaction utilisée est de préférence l'hydrogénation catalytique hétérogène ou le transfert de phase par le cyclohexène. Le solvant est un solvant protique ou aprotique et de préférence un alcool de point d'ébullition compris entre 66°C et 160°C. Le catalyseur est classiquement le palladium sur charbon. La réaction d'hydrogénation est généralement réalisée à une température comprise entre 25°C et 80°C sous une pression d'hydrogène comprise entre 1 bar et 40 bars, de préférence entre 1 bar et 8 bars.

La présente invention a de plus pour objet les composés nitro de formule (II) 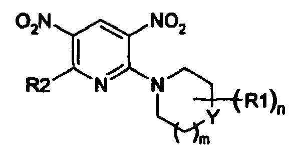 telle que définie à la présente revendication 28.

Les exemples qui suivent servent à illustrer l'invention sans toutefois présenter un caractère limitatif.

### EXEMPLES DE SYNTHESE :

### Exemple 1 : Dichlorhydrate de N-(3,5-diaminopyridin-2-yl)pyrrolidine, monohydrate

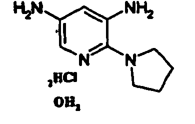

### Protocole A :

### Synthèse de la 3,5-Dinitro-2-pyrrolidin-1-yl-pyridine

Dans une solution contenant 3g ( 0,015 moles) de 2-chloro-3,5-dinitropyridine dans 30 ml de dioxane, à 40°C sont ajoutés 2,2 g ( 0,03 moles) de pyrrolidine en 10 minutes.
Le milieu réactionnel est maintenu à 60°C jusqu'à disparition des réactifs. Le milieu réactionnel est ensuite versé sur un mélange eau/glace sous vive agitation, le précipité est essoré, lavé à l'eau, puis séché jusqu'à poids constant pour conduire à 3,2 g de 3,5-Dinitro-2-pyrrolidin-1-yl-pyridine.
L'analyse en spectrométrie de masse et RMN sont conformes.

### Protocole B :

### - Synthèse du N-(3,5-diaminopyridin-2-yl)pyrrolidine, x HCl, y H₂O, z ROH :

4 g (0,0168 mole) de 3,5-Dinitro-2-(pyrrolidin-1-yl)-pyridine, obtenu selon le Protocole A à partir de 2-chloro-3,5-dinitropyridine et de pyrrolidine, sont réduits dans un autoclave dans 100 ml d'éthanol en présence de 10% de palladium sur charbon sous une pression de 8 Bars à température ambiante. Après disparition des réactifs, le catalyseur est éliminé par filtration, le filtrat est acidifié par de l'acide chlorhydrique et le dérivé réduit est isolé sous forme de di-chlorhydrates monohydrate.
Après séchage, on obtient une quantité de solide de 3,7 g.

| Analyse élémentaire du dichlorhydrate monohydrate | | | | |
|---|---|---|---|---|
| théorique | C: 40,16 | H : 6,74 | N : 20,81 | Cl : 26,34 |
| trouvée | C : 40,43 | H : 6,30 | N : 20,34 | Cl : 26,82 |

### Exemple 2 : synthèse du dichrohydrate de N-(3,5-diaminopyridin-2-yl)-3-hydroxypyrrolidine :

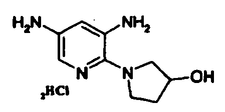

2 g ( 0,01 mole) de 3,5-dinitro-2-(3-hydroxypyrrolidin-1-yl)-pyridine obtenus selon le Protocole A à partir de 2-chloro-3,5-dinitropyridine et de 3-hydroxypyrrolidine, sont réduits selon le Protocole B, on obtient 1,1 g de dichrorhydrate de N-(3,5-diaminopyridin-2-yl)-3-hydroxypyrrolidine.
Après séchage, on obtient une quantité de solide de 0,9 g.
Les Analyses RMN et spectrométrie de masse sont conformes à la structure attendue.

### Exemple 3 : synthèse du N-(3,5-diaminopyridin-2-yl)-2-méthylpyrrolidine, 1,8 HCl, 1 MeOH :

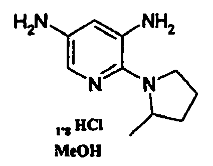

1,5 g ( 5,94 mmole) de (2-Methyl-pyrrolidin-1-yl)-3,5-dinitro-pyridine obtenus selon le Protocole A à partir de 2-chloro-3,5-dinitropyridine et de 2-méthylpyrrolidine, sont réduits par transfert d'hydrogène dans 50 ml d'éthanol en présence de palladium sur charbon et 5 ml de cyclohexène. Après filtration du catalyseur, Le sel de chlorhydrate est isolé en utilisant, lors du traitement, une solution d'acide chlorhydrique méthanolique. On obtient le N-(3,5-diaminopyridin-2-yl)-(2-méthyl)-pyrrolidine, 1,8 HCl, **1 MeOH.**

Après séchage jusqu'à poids constant, on obtient une quantité de solide de 890 mg.

| Analyse élémentaire théorique avec 1,8 moles d'acide chlorhydrique et une mole de méthanol | | | | |
|---|---|---|---|---|
| théorique | C : 45,56 | H : 7,52 | N : 19,33 | Cl : 22,07 |
| trouvée | C : 45.99 | H : 7.25 | N : 19,00 | Cl : 22.92 |

### Exemple 4 : synthèse du dichlorhydrate de N-(3,5-diaminopyridin-2-yl)-2,5-diméthylpyrrolidine :

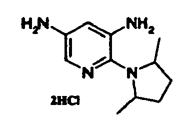

1,5 g ( 5,63 mmoles) de (2,5-diméthyl-pyrrolidin-1-yl)-3,5-dinitro-pyridine obtenus selon le Protocole A à partir de 2-chloro-3,5-dinitropyridine et de 2,5-diméthylpyrrolidine sont réduits par transfert d'hydrogène dans 50 ml d'éthanol en présence de palladium sur charbon et 5 ml de cyclohexène. On obtient 900mg de dichlorhydrate de N-(3,5-diaminopyridin-2-yl)-2,5-diméthylpyrrolidine.

Les Analyses RMN et spectrométrie de masse sont conformes à la structure attendue.

### Exemple 5 : synthèse du monohydrate de N-(3,5-diaminopyridin-2-yl)pipéridine, 1,9HCl

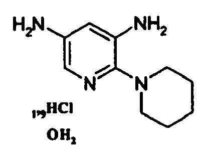

2 g de (pipéridin-1-yl)-3,5-dinitro-pyridine obtenus selon le Protocole A à partir de 2-chloro-3,5-dinitropyridine et de pipéridine, sont réduits par transfert d'hydrogène dans 50 ml d'éthanol en présence de palladium sur charbon et 5 ml de cyclohexène. 930mg de N-(3,5-diaminopyridin-2-yl)pipéridine , di-chlorhydrates sont ainsi obtenus.

| Analyse élémentaire théorique du monohydrate et avec 1,9 HCl : | | | | |
|---|---|---|---|---|
| théorique : | C : 42,96 | H : 7,12 | N : 20,05 | Cl : 24,14 |
| trouvée | C: 43,74 | H : 7, 12 | N : 19, 57 | Cl : 23,99 |

### Exemple 6 : Synthèse du dichlorhydrate de 2-(4-Methyl-piperazin-1-yl)-pyridine-3,5-diamine

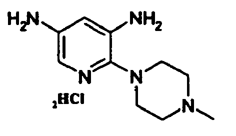

2 g de1-(3,5-Dinitro-pyridin-2-yl)-4-méthyl-pipérazine obtenus selon le Protocole A à partir de 2-chloro-3,5-dinitropyridine et de 4-méthylpipérazine, sont réduits par transfert d'hydrogène dans 50 ml d'éthanol en présence de palladium sur charbon et 5 ml de cyclohexène. 1,63 g de dichlorhydrate de 2-(4-méthyl-pipérazin-1-yl)-pyridine-3,5-diamine sont obtenus.

Les analyses RMN et spectrométrie de masse sont conformes à la structure attendue.

### Exemple 7 : Synthèse du trichlorhydrate de (4-éthylpipérazine-1-yl)pyridine-3,5-diamine

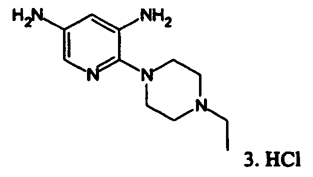

### A) Synthèse du (4-éthylpipérazine-1-yl)-3,5-dinitropyridine

Dans un ballon, on charge 3g (14,7 mmoles) de produit 2-chloro-3,5-dinitropyridine, 20ml de THF et 30 mmoles 4-éthylpipérazine. Le mélange est porté à 60°C pendant deux heures sous agitation puis le mélange est versé sur un mélange de glace eau sous agitation. Le précipité formé est essoré et séché sous vide jusqu'à poids constant. On obtient 5 g de poudre jaune.

Les analyses RMN et spectrométrie de masse sont conformes à la structure attendue.

### B) Synthèse du trichlorhydrate de (4-éthylpipérazine-1-yl)pyridine-3,5-diamine

Dans un ballon tout équipé, on charge 0,8g (2,8 mmoles) de (4-éthylpipérazine-1-yl) - 3.5-dinitropyridine synthétisé selon le mode opératoire (A) ci-dessus, 10ml d'éthanol, 2 ml de cyclohexène et 0,5 g de palladium sur charbon. Le mélange est porté au reflux pendant deux heures sous agitation puis le catalyseur est éliminé par filtration puis le filtrat est acidifié par de l'acide chlorhydrique. Après dilution à l'éther diisopropylique le précipité formé est essoré et séché sous vide jusqu'à poids constant. On obtient 0,41g de poudre.

Les analyses RMN et spectrométrie de masse sont conformes à la structure attendue.
**Analyse élémentaire :**
C 36.87% / H 7.05% / N 19.73% / Cl 29.41% / O 5.69%

### Exemple 8 : Synthèse du dichlorhydrate de 2-(3,5-dimethylpiperidin-1-yl)pyridine-3.5-diamine

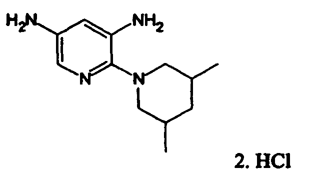

### A) Synthèse du 2-(3,5-dimethylpiperidin-1-yl)-3,5-dinitropyridine

Dans un ballon, on charge 2g (9,82 mmoles) de produit 2-chloro-3,5-dinitropyridine, 10ml de THF et 20 mmoles de 3,5-dimethylpiperidine. Le mélange est porté à 60°C pendant 15 heures sous agitation puis le mélange est versé sur un mélange de glace eau sous agitation. Le précipité formé est essoré et séché sous vide jusqu'à poids constant. On obtient 2,55 g de poudre jaune.

Les analyses RMN et spectrométrie de masse sont conformes à la structure attendue.

### B) Synthèse du dichlorhydrate de 2-(3,5-dimethylpiperidin-1-yl)pyridine-3,5-diamine

Dans un ballon tout équipé, on charge 1,27g (7 mmoles) de 2-(3.5-dimethylpiperidin-1-yl)-3.5-dinitropyridine synthétisé selon le mode opératoire (A) ci-dessus, 20ml d'éthanol, 5 ml de cyclohexène et 1 g de palladium sur charbon. Le mélange est porté au reflux pendant deux heures sous agitation puis le catalyseur est éliminé par filtration puis le filtrat est acidifié par de l'acide chlorhydrique. Après dilution à l'éther diisopropylique le précipité formé est essoré et séché sous vide jusqu'à poids constant. On obtient 1,32g de poudre.

Les analyses RMN et spectrométrie de masse sont conformes à la structure attendue.
**Analyse élémentaire :**
C 48.71% / H 7.92% / N18.96% / Cl 23.08% / O 3.03%

### Exemple 9 : Synthèse du dichlorhydrate de 2-(1-(3.5-diaminopyridine-2-yl)piperidine-2-yl) éthanol

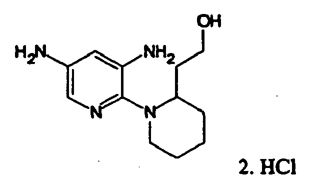

### A) Synthèse du 2-(1-(3.5-dinitropyridine-2-yl)piperidine-2-yl) éthanol

Dans un ballon, on charge 3g (14,7 mmoles) de produit 2-chloro-3,5-dinitropyridine, 10ml de THF et 30 mmoles de piperidine-2-éthanol. Le mélange est porté à 60°C pendant 15 heures sous agitation puis le mélange est versé sur un mélange de glace eau sous agitation. Le précipité formé est essoré et séché sous vide jusqu'à poids constant. On obtient 3,71 g de poudre jaune.

Les analyses RMN et spectrométrie de masse sont conformes à la structure attendue.

### B) Synthèse du dichlorhydrate de 2-(1-(3,5-diaminopyridine-2-yl)piperidine-2-yl) éthanol

Dans un ballon tout équipé, on charge 1,95g (6,6 mmoles) 2-(1-(3.5-dinitropyridine-2-yl)piperidine-2-yl) éthanol synthétisé selon le mode opératoire (A) ci-dessus, 20ml d'éthanol, 5 ml de cyclohexène et 1,5 g de palladium sur charbon. Le mélange est porté au reflux pendant deux heures sous agitation puis le catalyseur est éliminé par filtration puis le filtrat est acidifié par de l'acide chlorhydrique. Après dilution à l'éther diisopropylique le précipité formé est essoré et séché sous vide jusqu'à poids constant. On obtient 1,55g de poudre.

Les analyses RMN et spectrométrie de masse sont conformes à la structure attendue:
**Analyse élémentaire :**
C 44.27% / H 7.6% / N 16.34% / CI 22.16% / O 10.30%

### Exemple 10 : Synthèse du dichlorhydrate de 1-(3.5-diaminopyridine-2-yl)pipéridine-4-carboxamide

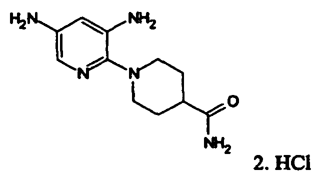

### A) Synthèse du 1-(3.5-nitropyridine-2-yl)pipéridine-4-carboxamide

Dans un ballon, on charge 3g (14,7 mmoles) de produit 2-chloro-3,5-dinitropyridine, 10ml de THF et 30 mmoles de pipéridine-4-carboxamide. Le mélange est porté à 60°C pendant 15 heures sous agitation puis le mélange est versé sur un mélange de glace eau sous agitation. Le précipité formé est essoré et séché sous vide jusqu'à poids constant. On obtient 0,522 g de poudre jaune.

Les analyses RMN et spectrométrie de masse sont conformes à la structure attendue.

### B) Synthèse du dichlorhydrate de 1-(3,5-diaminopyridine-2-yl)pipéridine-4-carboxamide

Dans un ballon tout équipé, on charge 1,93g (6,5 mmoles) 1-(3.5-nitropyridine-2-yl)pipéridine-4-carboxamide synthétisé selon le mode opératoire (A) ci-dessus, 20ml d'éthanol, 5 ml de cyclohexène et 1,5 g de palladium sur charbon. Le mélange est porté au reflux pendant deux heures sous agitation puis le catalyseur est éliminé par filtration puis le filtrat est acidifié par de l'acide chlorhydrique. Après dilution à l'éther diisopropylique le précipité formé est essoré et séché sous vide jusqu'à poids constant. On obtient 1,2g de poudre.

Les analyses RMN et spectrométrie de masse sont conformes à la structure attendue.
**Analyse élémentaire :**
C 40.94% / H 6.97% / N 21.23% / CI 21.47% / O 10.77%

### Exemple 11 : Synthèse du dichlorhydrate de 2-(2-méthylpipéridine-1-yl)pyridine-3.5-diamine

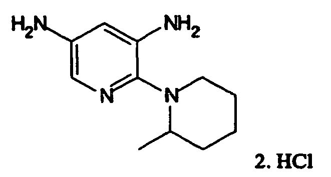

### A) Synthèse du 2-(2-méthylpipéridine-1-yl) -3.5-dinitropyridine

Dans un ballon, on charge 2,5g (12,28 mmoles) de produit 2-chloro-3,5-dinitropyridine, 10ml de THF et 25 mmoles de 2-méthylpipéridine. Le mélange est porté à 60°C pendant 15 heures sous agitation puis le mélange est versé sur un mélange de glace eau sous agitation. Le précipité formé est essoré et séché sous vide jusqu'à poids constant. On obtient 3,09 g de poudre jaune.

Les analyses RMN et spectrométrie de masse sont conformes à la structure attendue.

### B) Synthèse du dichlorhydrate de 2-(2-méthylpipéridine-1-yl)pyridine-3.5-diamine

Dans un ballon tout équipé, on charge 1,72g (6,5 mmoles) 2-(2-méthylpipéridine-1-yl) - 3.5-dinitropyridine synthétisé selon le mode opératoire (A) ci-dessus, 20ml d'éthanol, 5 ml de cyclohexène et 1,5 g de palladium sur charbon. Le mélange est porté au reflux pendant deux heures sous agitation puis le catalyseur est éliminé par filtration puis le filtrat est acidifié par de l'acide chlorhydrique. Après dilution à l'éther diisopropylique le précipité formé est essoré et séché sous vide jusqu'à poids constant. On obtient 1,24g de poudre.

Les analyses RMN et spectrométrie de masse sont conformes à la structure attendue.
**Analyse élémentaire:**
C 45.34% / H 7.47% / N 18.71% / Cl 22.01% / O 5.56%

### Exemple 12 : Synthèse du dichlorhydrate de 2-azépan-1-yt pyridine-3,5-diamine

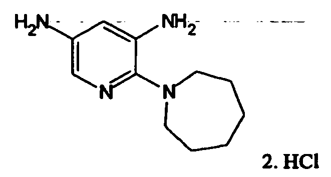

### A) Synthèse du 2-azépan-1-yl-3,5-dinitropyridine

Dans un ballon, on charge 3g (14,7 mmoles) de produit 2-chloro-3,5-dinitropyridine, 10ml de THF et 30 mmoles d'azépane. Le mélange est porté à 60°C pendant 15 heures sous agitation puis le mélange est versé sur un mélange de glace eau sous agitation. Le précipité formé est essoré et séché sous vide jusqu'à poids constant. On obtient 1,64 g de poudre jaune.

Les analyses RMN et spectrométrie de masse sont conformes à la structure attendue.

### B) Synthèse du dichlorhydrate de 2-azépan-1-yl pyridine-3,5-diamine

Dans un ballon tout équipé, on charge 1,66g (6,25 mmoles) 2-azépan-1-yl-3,5-dinitropyridine synthétisé selon le mode opératoire (A) ci-dessus, 20ml d'éthanol, 5 ml de cyclohexène et 1,5 g de palladium sur charbon. Le mélange est porté au reflux pendant deux heures sous agitation puis le catalyseur est éliminé par filtration puis le filtrat est acidifié par de l'acide chlorhydrique. Après dilution à l'éther diisopropylique le précipité formé est essoré et séché sous vide jusqu'à poids constant. On obtient 1,03g de poudre.

Les analyses RMN et spectrométrie de masse sont conformes à la structure attendue.
**Analyse élémentaire :**
C 40.46% / H 6.88% / N 18.47% / Cl 23.37% / O 9.74%

### Exemple 13 : Synthèse du dichlorhydrate de 2-moroholin-4-yl pyridine-3.5-diamine

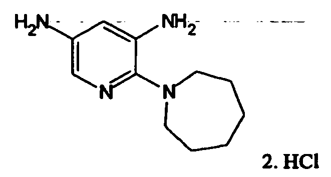

### A) Synthèse du 2-morpholin-4-yl-3,5-dinitropyridine

Dans un ballon, on charge 5g (24,5 mmoles) de produit 2-chloro-3,5-dinitropyridine, 40ml de THF et 50 mmoles de morpholine. Le mélange est porté à 60°C pendant 15 heures sous agitation puis le mélange est versé sur un mélange de glace eau sous agitation. Le précipité formé est essoré et séché sous vide jusqu'à poids constant. On obtient 6,09 g de poudre jaune.

Les analyses RMN et spectrométrie de masse sont conformes à la structure attendue.

### B) Synthèse du dichlorhydrate de 2-morpholin-4-yl pyridine-3.5-diamine

Dans un ballon tout équipé, on charge 3,5g (13,76 mmoles) de 2-morpholin-4-yl-3.5-dinitropyridine synthétisé selon le mode opératoire (A) ci-dessus, 20ml d'éthanol, 5 ml de cyclohexène et 2 g de palladium sur charbon. Le mélange est porté au reflux pendant deux heures sous agitation puis le catalyseur est éliminé par filtration puis le filtrat est acidifié par de l'acide chlorhydrique. Après dilution à l'éther diisopropylique le précipité formé est essoré et séché sous vide jusqu'à poids constant. On obtient 0,98g de poudre.

Les analyses RMN et spectrométrie de masse sont conformes à la structure attendue.

### EXEMPLES DE TEINTURE

### EXEMPLES A : exemples 1 à 24 de teinture en milieu alcalin

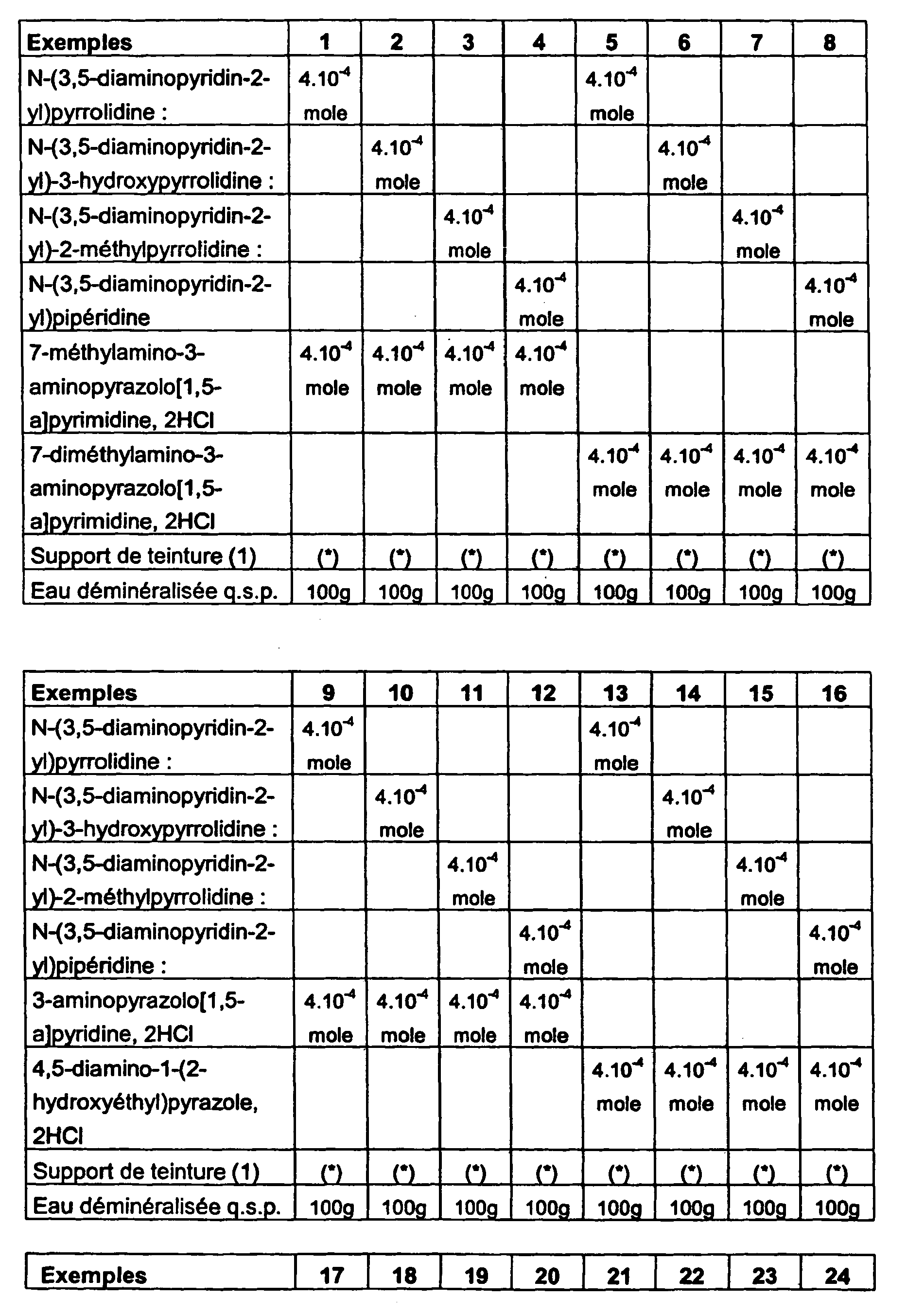

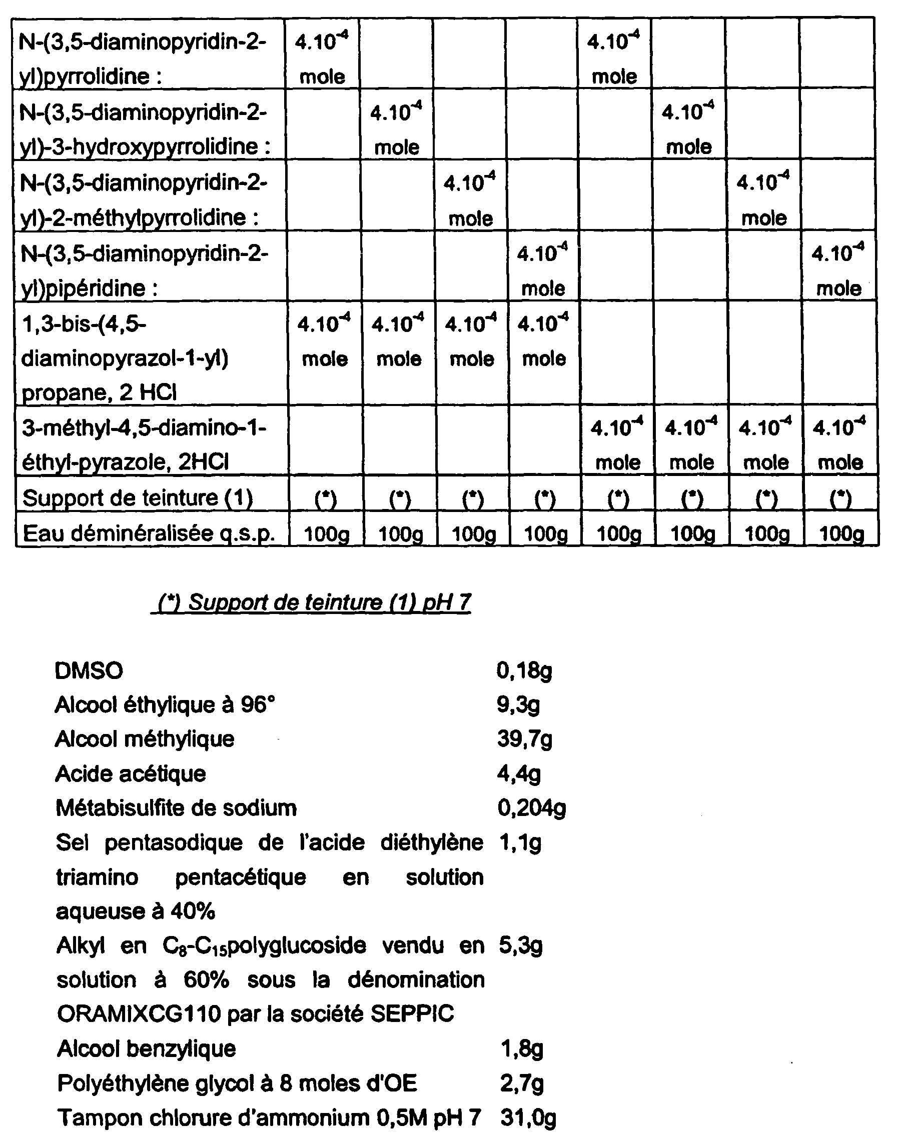

Au moment de l'emploi, chaque composition est mélangée avec un tiers de son poids d'eau oxygénée à 20 volumes (6% en poids).

Chaque mélange obtenu est appliqué sur une mèche de cheveux gris à 90 % de blancs. Après 30 min de pose, les mèches sont rincées, lavées avec un shampooing standard, rincées à nouveau puis séchées.

Les résultats de teinture suivants ont été obtenus.

| **Exemple** | **1.** | **2** | **3** | **4** | **5** | **6** | **7** | **8** |
|---|---|---|---|---|---|---|---|---|
| **Nuance** | Gris | violet | Gris | Gris | Gris | gris | Gris | Gris |
| **observée** | | foncé | | | | bleu | | |

| **Exemple** | **9** | **10** | **11** | **12** | **13** | **14** | **15** | **16** |
|---|---|---|---|---|---|---|---|---|
| **Nuance** | Gris | Gris | Gris | Gris | Gris | gris | violet | violet |
| **observée** | violacé | violacé | violacé | violacé | | | | bleu |

| **Exemple** | **17** | **18** | **19** | **20** | **21** | **22** | **23** | **24** |
|---|---|---|---|---|---|---|---|---|
| **Nuance** | gris | gris | Gris | Gris | Gris | Gris | Gris | Gris |
| **observée** | violacé - bleuté | bleu | violacé | clair | violacé | violacé | | |

### EXEMPLES B DE TEINTURE EN MILIEU ALCALIN

### Exemple 1 à 7

On a préparé les compositions tinctoriales suivantes :

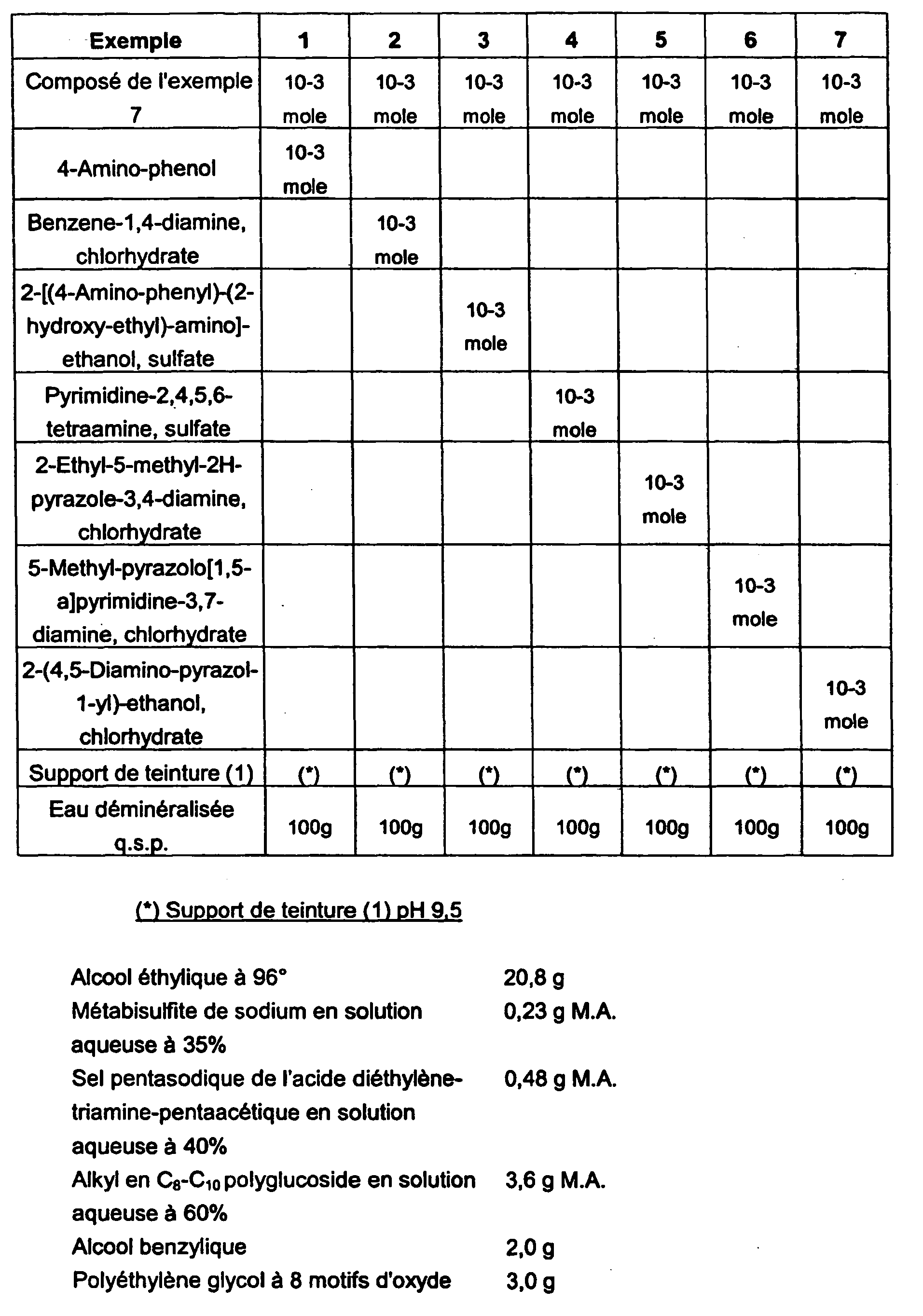

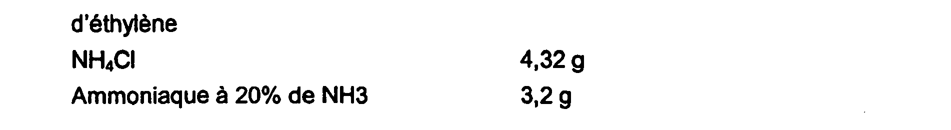

Au moment de l'emploi, chaque composition est mélangée avec un poids égal d'eau oxygénée à 20 volumes (6% en poids). On obtient un pH final de 9,5.

Chaque mélange obtenu est appliqué sur des mèches de cheveux gris à 90 % de blancs. Après 30 min de pose, les mèches sont rincées, lavées avec un shampooing standard, rincées à nouveau puis séchées. Les résultats de teinture suivants ont été obtenus :

| **Exemple** | **1** | **2** | **3** | **4** | **5** | **6** | **7** |
|---|---|---|---|---|---|---|---|
| **Nuance observée** | orangé | gris intense | vert-bleu intense | gris intense | gris violet intense | violet intense | gris violet intense |

### Exemples 8 à 9

On a préparé les compositions tinctoriales suivantes :

| **Exemple** | **8** | **9** |
|---|---|---|
| Composé de l'exemple 13 | 10-3 mole | 10-3 mole |
| 2-[(4-Amino-phenyl)-(2-hydroxy-ethyl)amino]-ethanol, sulfate | 10-3 mole | |
| Pyrimidine-2,4,5,6-tetraamine, sulfate | | 10-3 mole |
| Support de teinture (1) | (*) | (*) |
| Eau déminéralisée q.s.p. | 100g | 100g |

Voir la composition du support de teinture (1) pH 9,5 et l'application dans les exemples 1 à 7.

Les résultats de teinture suivants ont été obtenus :

| **Exemple** | **8** | **9** |
|---|---|---|
| **Nuance observée** | vert-bleu intense | gris violet intense |

### Exemples 10 à 15

On a préparé les compositions tinctoriales suivantes :

| **Exemple** | **10** | **11** | **12** | **13** | **14** | **15** |
|---|---|---|---|---|---|---|
| Composé de l'exemple 11 | 10-3 mole | 10-3 mole | 10-3 mole | 10-3 mole | 10-3 mole | 10-3 mole |
| 4-Amino-phenol | 10-3 mole | | | | | |
| Benzene-1,4-diamine, chlorhydrate | | 10-3 mole | | | | |
| 2-[(4-Amino-phenyl)-(2-hydroxy-ethyl)-amino]-ethanol, sulfate | | | 10-3 mole | | | |
| Pyrimidine-2,4,5,6-tetraamine, sulfate | | | | 10-3 mole | | |
| 5-Methyl-pyrazolo[1,5-a]pyrimidine-3,7-diamine, chlorhydrate | | | | | 10-3 mole | |
| 2-(4,5-Diamino-pyrazol-1-yl)-ethanol, chlorhydrate | | | | | | 10-3 mole |
| Support de teinture (1) | (*) | (*) | (*) | (*) | (*) | (*) |
| Eau déminéralisée q.s.p. | 100g | 100g | 100g | 100g | 100g | 100g |

Voir la composition du support de teinture (1) pH 9,5 et l'application dans les exemples 1 à 7.

Les résultats de teinture suivants ont été obtenus :

| **Exemple** | **10** | **11** | **12** | **13** | **14** | **15** |
|---|---|---|---|---|---|---|
| **Nuance observée** | orangé | gris intense | vert intense | gris intense | gris violet intense | gris violet intense |

### Exemples 16 A 18

On a préparé les compositions tinctoriales suivantes :

| **Exemple** | **16** | **17** | **18** |
|---|---|---|---|
| Composé de l'exemple 12 | 10-3 mole | 10-3 mole | 10-3 mole |
| 2-[(4-Amino-phenyl)-(2-hydroxy-ethyl)-amino]-ethanol, sulfate | 10-3 mole | | |
| Pyrimidine-2,4,5,6-tetraamine, sulfate | | 10-3 mole | |
| 2-(4,5-Diamino-pyrazol-1-yl)-ethanol, chlorhydrate | | | 10-3 mole |
| Support de teinture (1) | (*) | (*) | (*) |
| Eau déminéralisée q.s.p. | 100g | 100g | 100g |

Voir la composition du support de teinture (1) pH 9,5 et l'application dans les exemples 1 à 7.

Les résultats de teinture suivants ont été obtenus :

| **Exemple** | **16** | **17** | **18** |
|---|---|---|---|
| **Nuance observée** | vert-bleu intense | gris intense | gris violet intense |

### Exemples 19 A 21

On a préparé les compositions tinctoriales suivantes :

| **Exemple** | **19** | **20** | **21** |
|---|---|---|---|
| Composé de l'exemple 8 | 10-3 mole | 10-3 mole | 10-3 mole |
| 2-[(4-Amino-phenyl)-(2-hydroxy-ethyl)-amino]-ethanol, sulfate | 10-3 mole | | |
| Pyrimidine-2,4,5,6-tetraamine, sulfate | | 10-3 mole | |
| 2-(4,5-Diamino-pyrazol-1-yl)- ethanol, chlorhydrate | | | 10-3 mole |
| Support de teinture (1) | (*) | (*) | (*) |
| Eau déminéralisée q.s.p. | 100g | 100g | 100g |

Voir la composition du support de teinture (1) pH 9,5 et l'application dans les exemples 1 à 7.

Les résultats de teinture suivants ont été obtenus :

| **Exemple** | **19** | **20** | **21** |
|---|---|---|---|
| **Nuance observée** | vert-bleu intense | gris vert-bleu intense | gris violet intense |

### Exemples 22 à 28

On a préparé les compositions tinctoriales suivantes :

| **Exemple** | **22** | **23** | **24** | **25** | **26** | **27** | **28** |
|---|---|---|---|---|---|---|---|
| Composé de l'exemple 9 | 10-3 mole | 10-3 mole | 10-3 mole | 10-3 mole | 10-3 mole | 10-3 mole | 10-3 mole |
| 4-Amino-phenol | 10-3 mole | | | | | | |
| Benzene-1,4-diamine, chlorhydrate | | 10-3 mole | | | | | |
| 2-[(4-Amino-phenyl)-(2-hydroxy-ethyl)-amino]-ethanol, sulfate | | | 10-3 mole | | | | |
| Pyrimidine-2,4,5,6-tetraamine, sulfate | | | | 10-3 mole | | | |
| 2-Ethyl-5-methyl-2H-pyrazole-3,4-diamine, chlorhydrate | | | | | 10-3 mole | | |
| 5-Methyl-pyrazolo[1,5-a]pyrimidine-3,7-diamine, chlorhydrate | | | | | | 10-3 mole | |
| 2-(4,5-Diamino-pyrazol-1-yl)-ethanol, chlorhydrate | | | | | | | 10-3 mole |
| Support de teinture (1) | (*) | (*) | (*) | (*) | (*) | (*) | (*) |
| Eau déminéralisée q.s.p. | 100g | 100g | 100g | 100g | 100g | 100g | 100g |

Voir la composition du support de teinture (1) pH 9,5 et l'application dans les exemples 1 à 7.

Les résultats de teinture suivants ont été obtenus :

| **Exemple** | **22** | **23** | **24** | **25** | **26** | **27** | **28** |
|---|---|---|---|---|---|---|---|
| **Nuance observée** | rouge | gris intense | intense | gris intense | intense | gris rouge intense | gris violet- rouge intense |

### Exemples 29 à 34

On a préparé les compositions tinctoriales suivantes :

| **Exemple** | **29** | **30** | **31** | **32** | **33** | **34** |
|---|---|---|---|---|---|---|
| Composé de l'exemple 10 | 10-3 mole | 10-3 mole | 10-3 mole | 10-3 mole | 10-3 mole | 10-3 mole |
| 4-Amino-phenol | 10-3 mole | | | | | |
| Benzene-1,4-diamine, chlorhydrate | | 10-3 mole | | | | |
| 2-[(4-Amino-phenyl)-(2-hydroxy-ethyl)-amino]-ethanol, sulfate | | | 10-3 mole | | | |
| Pyrimidine-2,4,5,6-tetraamine, sulfate | | | | 10-3 mole | | |
| 5-Methyl-pyrazolo[1,5-a]pyrimidine-3,7-diamine, chlorhydrate | | | | | 10-3 mole | |
| 2-(4,5-Diamino-pyrazol-1-yl)-ethanol, chlorhydrate | | | | | | 10-3 mole |
| Support de teinture (1) | (*) | (*) | (*) | (*) | (*) | (*) |
| Eau déminéralisée q.s.p. | 100g | 100g | 100g | 100g | 100g | 100g |

Voir la composition du support de teinture (1) pH 9,5 et l'application dans les exemples 1 à 7.

Les résultats de teinture suivants ont été obtenus :

| **Exemple** | **29** | **30** | **31** | **32** | **33** | **34** |
|---|---|---|---|---|---|---|
| **Nuance observée** | orangé | gris intense | vert-bleu intense | gris | gris violet intense | gris violet intense |

### EXEMPLES C DE TEINTURE EN MILIEU ACIDE

### Exemples 35 à 40

On a préparé les compositions tinctoriales suivantes :

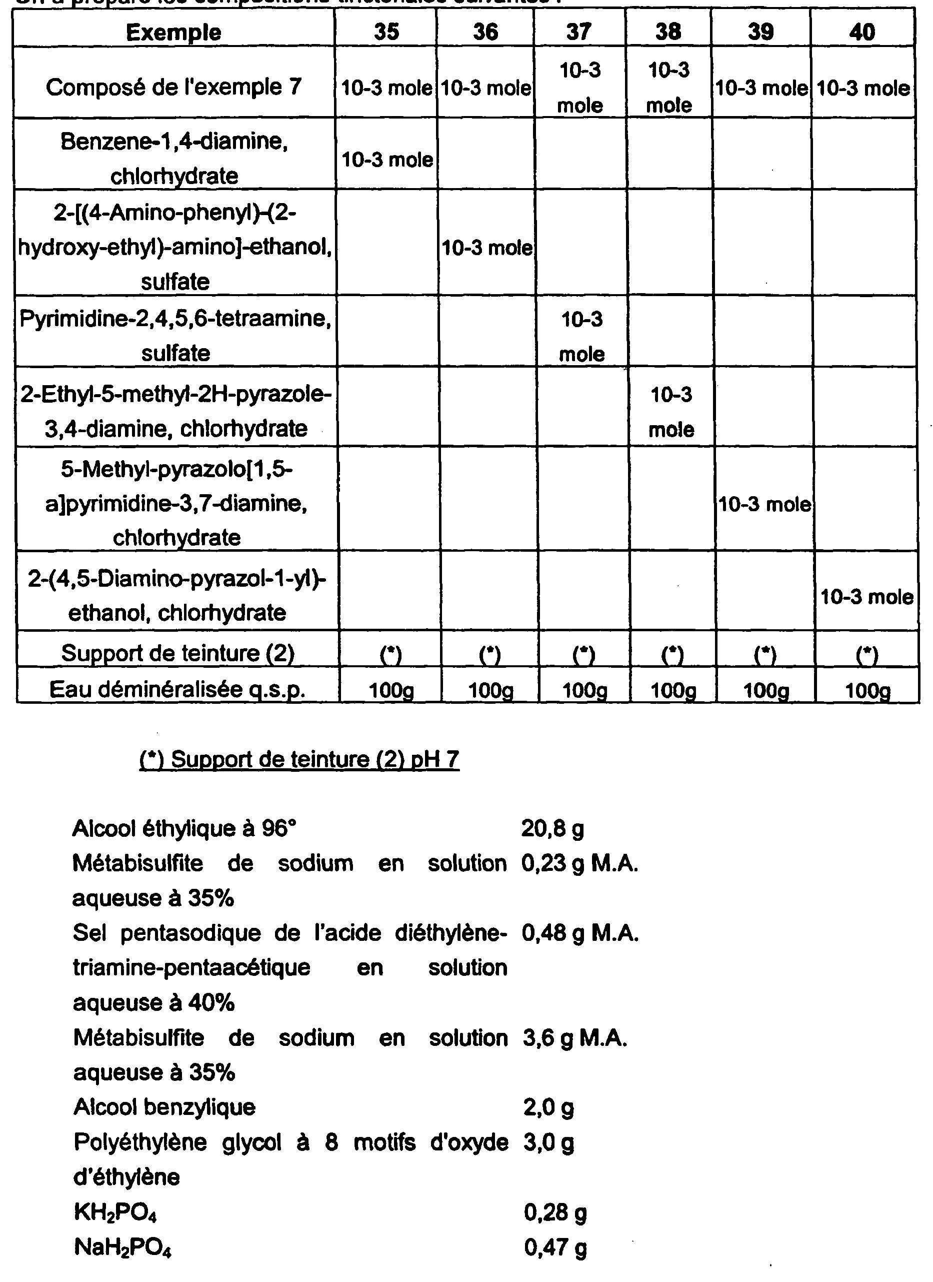

Au moment de l'emploi, chaque composition est mélangée avec un poids égal d'eau oxygénée à 20 volumes (6% en poids). On obtient un pH final de 7.

Chaque mélange obtenu est appliqué sur des mèches de cheveux gris à 90 % de blancs. Après 30 min de pose, les mèches sont rincées, lavées avec un shampooing standard, rincées à nouveau puis séchées.

Les résultats de teinture suivants ont été obtenus :

| **Exemple** | **35** | **36** | **37** | **38** | **39** | **40** |
|---|---|---|---|---|---|---|
| **Nuance observée** | gris vert-bleu intense | vert-bleu intense | gris violet- bleu intense | gris violet intense | violet intense | gris violet intense |

### Exemples 41 à 43

On a préparé les compositions tinctoriales suivantes :

| **Exemple** | **41** | **42** | **43** |
|---|---|---|---|
| Composé de l'exemple 13 | 10-3 mole | 10-3 mole | 10-3 mole |
| 4-Amino-phenol | 10-3 mole | | |
| 2-[(4-Amino-phenyl)-(2-hydroxy-ethyl)-amino]-ethanol, sulfate | | 10-3 mole | |
| Pyrimidine-2,4,5,6-tetraamine, sulfate | | | 10-3 mole |
| Support de teinture (2) | (*) | (*) | (*) |
| Eau déminéralisée q.s.p. | 100g | 100g | 100g |

Voir la composition du support de teinture (2) pH 7 et l'application dans les exemples 35 à 40.

Les résultats de teinture suivants ont été obtenus :

| **Exemple** | **41** | **42** | **43** |
|---|---|---|---|
| **Nuance observée** | brun orangé intense | gris vert-bleu intense | gris intense |

### Exemples 44 à 50

On a préparé les compositions tinctoriales suivantes :

| **Exemple** | **44** | **45** | **46** | **47** | **48** | **49** | **50** |
|---|---|---|---|---|---|---|---|
| Composé de l'exemple 11 | 10-3 mole | 10-3 mole | 10-3 mole | 10-3 mole | 10-3 mole | 10-3 mole | 10-3 mole |
| 4-Amino-phenol | 10-3 mole | | | | | | |
| Benzene-1,4-diamine, chlorhydrate | | 10-3 mole | | | | | |
| 2-[(4-Amino-phenyl)-(2-hydroxy-ethyl)-amino]-ethanol, sulfate | | | 10-3 Mole | | | | |
| Pyrimidine-2,4,5,6-tetraamine, sulfate | | | | 10-3 mole | | | |
| 2-Ethyl-5-methyl-2H-pyrazole-3,4-diamine, chlorhydrate | | | | | 10-3 mole | | |
| 5-Methyl-pyrazolo[1,5-a]pyrimidine-3,7-diamine, chlorhydrate | | | | | | 10-3 mole | |
| 2-(4,5-Diamino-pyrazol-1-yl)-ethanol, chlorhydrate | | | | | | | 10-3 mole |
| Support de teinture (2) | (*) | (*) | (*) | (*) | (*) | (*) | (*) |
| Eau déminéralisée q.s.p. | 100g | 100g | 100g | 100g | 100g | 100g | 100g |

Voir la composition du support de teinture (2) pH 7 et l'application dans les exemples 35 à 40.

Les résultats de teinture suivants ont été obtenus :

| **Exemple** | **44** | **45** | **46** | **47** | **48** | **49** | **50** |
|---|---|---|---|---|---|---|---|
| **Nuance observée** | orangé | gris intense | vert-bleu intense | gris intense | gris violet intense | gris violet intense | gris violet intense |

### Exemples 51 à 54

On a préparé les compositions tinctoriales suivantes :

| **Exemple** | **51** | **52** | **53** | **54** |
|---|---|---|---|---|
| Composé de l'exemple 12 | 10-3 mole | 10-3 mole | 10-3 mole | 10-3 mole |
| Benzene-1,4-diamine, chlorhydrate | 10-3 mole | | | |
| 2-[(4-Amino-phenyl)-(2-hydroxy-ethy l)-amino]-ethanol, sulfate | | 10-3 mole | | |
| Pyrimidine-2,4,5,6-tetraamine, sulfate | | | 10-3 mole | |
| 2-(4,5-Diamino-pyrazol-1-yl)-ethanol, chlorhydrate | | | | 10-3 mole |
| Support de teinture (2) | (*) | (*) | (*) | (*) |
| Eau déminéralisée q.s.p. | 100g | 100g | 100g | 100g |

Voir la composition du support de teinture (2) pH 7 et l'application dans les exemples 35 à 40.

Les résultats de teinture suivants ont été obtenus :

| **Exemple** | **51** | **52** | **53** | **54** |
|---|---|---|---|---|
| **Nuance observée** | gris intense | vert-bleu intense | gris intense | gris violet intense |

### Exemples 55 à 61

On a préparé les compositions tinctoriales suivantes :

| **Exemple** | **55** | **56** | **57** | **58** | **59** | **60** | **61** |
|---|---|---|---|---|---|---|---|
| Composé de l'exemple 8 | 10-3 mole | 10-3 mole | 10-3 mole | 10-3 mole | 10-3 mole | 10-3 mole | 10-3 mole |
| 4-Amino-phenol | 10-3 mole | | | | | | |
| Benzene-1,4-diamine, chlorhydrate | | 10-3 mole | | | | | |
| 2-[(4-Amino-phenyl)-(2-hydroxy-ethyl)-amino]-ethanol, sulfate | | | 10-3 mole | | | | |
| Pyrimidine-2,4,5,6-tetraamine, sulfate | | | | 10-3 mole | | | |
| 2-Ethyl-5-methyl-2H-pyrazole-3,4-diamine, chlorhydrate | | | | | 10-3 mole | | |
| 5-Methyl-pyrazolo[1,5-a]pyrimidine-3,7-diamine, chlorhydrate | | | | | | 10-3 mole | |
| 2-(4,5-Diamino-pyrazol-1-yl)-ethanol, chlorhydrate | | | | | | | 10-3 mole |
| Support de teinture (2) | (*) | (*) | (*) | (*) | (*) | (*) | (*) |
| Eau déminéralisée q.s.p. | 100g | 100g | 100g | 100g | 100g | 100g | 100g |

Voir la composition du support de teinture (2) pH 7 et l'application dans les exemples 35 à 40.
Les résultats de teinture suivants ont été obtenus :

| **Exemple** | **55** | **56** | **57** | **58** | **59** | **60** | **61** |
|---|---|---|---|---|---|---|---|
| **Nuance observée** | orangé | gris vert- bleu intense | vert-bleu intense | gris intense | gris intense | gris violet intense | gris violet intense |

### Exemples 62 à 68

On a préparé les compositions tinctoriales suivantes :

| **Exemple** | **62** | **63** | **64** | **65** | **66** | **67** | **68** |
|---|---|---|---|---|---|---|---|
| Composé de l'exemple 9 | 10-3 mole | 10-3 mole | 10-3 mole | 10-3 mole | 10-3 mole | 10-3 mole | 10-3 mole |
| 4-Amino-phenol | 10-3 mole | | | | | | |
| Benzene-1,4-diamine, chlorhydrate | | 10-3 mole | | | | | |
| 2-[(4-Amino-phenyl)-(2-hydroxy-ethyl)-amino]-ethanol, sulfate | | | 10-3 mole | | | | |
| Pyrimidine-2,4,5,6-tetraamine, sulfate | | | | 10-3 mole | | | |
| 2-Ethyl-5-methyl-2H-pyrazole-3,4-diamine, chlorhydrate | | | | | 10-3 mole | | |
| 5-Methyl-pyrazolo[1,5-a]pyrimidine-3,7-diamine, chlorhydrate | | | | | | 10-3 mole | |
| 2-(4,5-Diamino-pyrazol-1-yl)-ethanol, chlorhydrate | | | | | | | 10-3 mole |
| Support de teinture (2) | (*) | (*) | (*) | (*) | (*) | (*) | (*) |
| Eau déminéralisée q.s.p. | 100g | 100g | 100g | 100g | 100g | 100g | 100g |

Voir la composition du support de teinture (2) pH 7 et l'application dans les exemples 35 à 40.
Les résultats de teinture suivants ont été obtenus :

| **Exemple** | **62** | **63** | **64** | **65** | **66** | **67** | **68** |
|---|---|---|---|---|---|---|---|
| **Nuance observée** | orangé | gris intense | gris vert- bleu intense | gris intense | gris intense | gris violet-rouge intense | gris violet-rouge intense |

### Exemples 69 à 74

On a préparé les compositions tinctoriales suivantes:

| **Exemple** | **69** | **70** | **71** | **72** | **73** | **74** |
|---|---|---|---|---|---|---|
| Composé de l'exemple 10 | 10-3 mole | 10-3 mole | 10-3 mole | 10-3 mole | 10-3 mole | 10-3 mole |
| 4-Amino-phenol | 10-3 mole | | | | | |
| Benzene-1,4-diamine, chlorhydrate | | 10-3 mole | | | | |
| 2-[(4-Amino-phenyl)-(2-hydroxy-ethyl)-amino]-ethanol, sulfate | | | 10-3 mole | | | |
| Pyrimidine-2,4,5,6-tetraamine, sulfate | | | | 10-3 mole | | |
| 2-Ethyl-5-methyl-2H-pyrazole-3,4-diamine, chlorhydrate | | | | | 10-3 mole | |
| 2-(4,5-Diamino-pyrazol-1-yl)-ethanol, chlorhydrate | | | | | | 10-3 mole |
| Support de teinture (2) | (*) | (*) | (*) | (*) | (*) | (*) |
| Eau déminéralisée q.s.p. | 100g | 100g | 100g | 100g | 100g | 100g |

Voir la composition du support de teinture (2) pH 7 et l'application dans les exemples 35 à 40.

Les résultats de teinture suivants ont été obtenus :

| **Exemple** | **69** | **70** | **71** | **72** | **73** | **74** |
|---|---|---|---|---|---|---|
| **Nuance observée** | orangé | gris intense | vert-bleu intense | gris intense | gris intense | gris violet intense |

## Revendications

1. Composition tinctoriale comprenant, dans un milieu de teinture approprié :
- au moins une base d'oxydation, et
- au moins un coupleur 2,3,5-triaminopyridine de formule (I) ou l'un de ses sels d'addition correspondants : 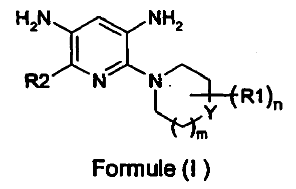
dans laquelle :
• R₁ représente:
- un atome d'halogène;
- un alkyle en C₁-C₄ éventuellement substitué par un ou plusieurs radicaux choisis parmi les radicaux hydroxy, carboxy, alcoxycarbonyle en C₁-C₄, carboxamido, alkyl(C₁-C₄)sulfonyle, alcoxy en C₁-C₄, alkyl(C₁-C₄)sulfonamido, NR₃R₄ ;
- un radical carboxy ;
- un radical alcoxycarbonyle en C₁-C₄ ;
- un radical carboxamido ;
- un radical alkyl en C₁-C₄ carboxamido ;
- un radical sulfinique
- un radical alkyl(C₁-C₄)sulfonyle ;
- un radical alkyl(C₁-C₄)sulfonamido ;
- un radical hydroxy ;
- un radical alcoxy en C₁-C₄ ;
- un radical hydroxyalcoxy en C₂-C₄ ;
- un radical amino ou mono- ou di- amino alcoxy;
- un radical thioéther en C₁-C₄ ;
- un radical alkyl(C₁-C₄)sulfoxyde ;;
- un radical sulfonique ;
- un radical NR₅R₆ ;
• R₃, R₄, R₅ et R₆ représentent, identiques ou différents, un atome d'hydrogène ; un radical alkyl(C₁-C₄)sulfonyle ; un radical alkyle(C₁-C₄)carbonyle dans lequel le radical alkyle peut être substitué par un ou plusieurs hydroxy ; un radical arylcarbonyle, le radical aryle pouvant être substitué par un radical choisi parmi hydroxy, alcoxy en C₁-C₄, amino ou (di)alkyl(C₁-C₄)amino ; un radical carboxamido ; un radical alkyle en C₁-C₄ éventuellement substitué par un ou plusieurs radicaux choisis parmi les radicaux hydroxy, alcoxy en C₁-C₄, alkyl(C₁-C₄)sulfonyle, alkyl(C₁-C₄)sulfonamido, carboxy, carboxamido, alkyl(C₁-C₄)sulfoxyde, amino , (di)(C₁-C₄)alkylamino ou (poly)hydroxyalkylamino en C₂-C₄ ;
• R₂ représente un atome d'hydrogène; un radical alcoxy en C₁-C₄ éventuellement substitué par un ou plusieurs radicaux hydroxy, alcoxy en C₁-C₂,
• n est un nombre entier compris entre 0 et 7, inclus
• m est 0, 1 ou 2,
• Y représente un atome d'oxygène, un radical C(R₈)₂ ou un radical NR₇ où R₇ a la même signification que R₃ et R₈, identiques ou différents, représentent un atome d'hydrogène ou ont la même signification que R₁.

2. Composition selon la revendication 1 dans laquelle R₁ représente un radical alcoxy en C1-C4 éventuellement substitué par un ou plusieurs radicaux hydroxy, alcoxy en C1-C2, amino ou (di)alkylamino; un radical hydroxy ; un radical amino ; un radical (di)alkylamino ; un radical alkyle en C1-C2 éventuellement substitué par un hydroxy, amino.

3. Composition selon la revendication 1 ou 2 dans laquelle R₂ représente un atome d'hydrogène ou un radical alcoxy.

4. Composition selon l'une quelconque des revendications 1 à 4 dans laquelle n représente 0 ou 1.

5. Composition selon l'une quelconque des revendications 1 à 4 dans laquelle R₃, R₄, R₅, R₆ et R₇ représentent, identiques ou différents, un atome d'hydrogène, un radical carboxamido, un radical alkyle en C₁-C₄ éventuellement substitué par un ou plusieurs radicaux choisis parmi les radicaux hydroxy, alcoxy en C₁-C₄, carboxamido, amino, (di)(C₁-C₄)alkylamino, (poly)hydroxyalkylamino en C₂-C₄.

6. Composition selon la revendication 1 dans laquelle R₃, R₄, R₅, R₆ et R₇ représentent, identiques ou différents, un atome d'hydrogène, un radical méthyle, un éthyle, 2-carboxyéthyle, 2-hydroxyéthyle, 3-hydroxypropyle, 2,3-dihydroxypropyle, 2-hydroxy-3-aminopropyle, 3-hydroxy-2-aminopropyle.

7. Composition selon la revendication 6 dans laquelle R₃, R₄, R₅, R₆ et R₇ représentent, identiques ou différents un atome d'hydrogène, un radical méthyle, un radical 2-hydroxyéthyle, un radical 2,3-dihydroxypropyle.

8. Composition selon l'une quelconque des revendications 1 à 7 dans laquelle l'azote en position 2 du cycle, Y et m forment un radical hétérocyclique choisi parmi les pyrrolidines, les pipéridines, les homopipéridines, les pipérazines, les homopipérazines, les diazépanes.

9. Composition selon la revendication 8 dans laquelle l'hétérocycle est choisi parmi la pyrrolidine, la 2,5-diméthylpyrrolidine, la 2-méthylpyrrolidine la proline, la 3-hydroxyproline, la 4-hydroxyproline, la 2,4-dicarboxypyrrolidine, la 2-hydroxyméthylpyrrolidine, la 3-hydroxy-2-hydroxyméthylpyrrolidine, la 2,5-di(hydroxyméthyl)pyrrolidine, la 2-carboxamidopyrrolidine, la 3-hydroxy-2-carboxamidopyrrolidine, la 2-(diméthylcarboxamido)pyrrolidine, la 2-(diméthylcarboxamido)-3-hydroxy-pyrrolidine, la 3,4-dihydroxy-2-hydroxyméthyl pyrrolidine, la 3-hydroxypyrrolidine, la 3,4-dihydroxypyrrolidine, la 3-aminopyrrolidine, la 3-méthylaminopyrrolidine, la 3-diméthylaminopyrrolidine, la 4-amino-3-hydroxy pyrrolidine, la 4-méthylamino-3-hydroxypyrrolidine, la 3-hydroxy-4-(2-hydroxyéthyl)amino-pyrrolidine, la pipéridine, la 2,6-diméthylpipéridine, la 2-carboxypipéridine, la 2-carboxamidopipéridine, la 2-(diméthylcarboxamido)pipéridine, la 2-hydroxyméthylpipéridine, la 3-hydroxy-2-hydroxyméthylpipéridine, 3-hydroxypipéridine, la 4-hydroxypipéridine, la 3-hydroxyméthylpipéridine, l'homopipéridine, la 2-carboxyhomopipéridine, la 2-carboxamidohomopipéridine, la pipérazine, la 4-méthylpipérazine le diazépane, N-méthyl homopipérazine, la N β-hydroxyéthylhomopipérazine et leurs sels d'addition.

10. Composition selon la revendication 8 dans laquelle l'hétérocycle est choisi parmi la pyrrolidine, la 2-méthylpyrrolidine, la 3-hydroxypyrrolidine, la 3-aminopyrrolidine, la 3-(méthylsulfonylamino)-pyrrolidine, la proline, la 3-hydroxyproline, la pipéridine, l'hydroxypipéridine, l'homopipéridine, la 4-méthylpipérazine, le diazépane, N-méthyl homopipérazine, la N β-hydroxyéthylhomopipérazine et leurs sels d'addition.

11. Composition selon la revendication 10 dans laquelle l'hétérocycle est choisi parmi la pyrrolidine, la 3-hydroxypyrrolidine, la 3-aminopyrrolidine, la 3-(méthylsulfonylamino)-pyrrolidine, la proline, la 3-hydroxyproline.

12. Composition selon la revendication 1 dans laquelle R1 représente un radical alkyle, amino, hydroxyalkyle, hydroxy, R2 est l'hydrogène, m est 0 ou 1, et n est compris entre 0, 1 ou 2.

13. Composition selon l'une quelconque des revendications 1 à 12 dans laquelle les composés de formule (I) sont choisis parmi :
N-(3,5-diaminopyridin-2-yl)pyrrolidine
N-(3,5-diaminopyridin-2-yl)-2-méthylpyrrolidine
N-(3,5-diaminopyridin-2-yl)-2,5-diméthylpyrrolidine
N-(3,5-diaminopyridin-2-yl)-2-hydroxyméthylpyrrolidine
N-(3,5-diaminopyridin-2-yl)proline
N-(3,5-diaminopyridin-2-yl)-3-hydroxypyrrolidine
N-(3,5-diaminopyridin-2-yl)-2-carboxamidopyrrolidine
N-(3,5-diaminopyridin-2-yl)-2-diméthylcarboxamidopyrrolidine
N-(3,5-diaminopyridin-2-yl)-3,4-dihydroxypyrrolidine
N-(3,5-diaminopyridin-2-yl)-3-aminopyrrolidine
N-(3,5-diaminopyridin-2-yl)-3-diméthylaminopyrrolidine
N-(3,5-diaminopyridin-2-yl)pipéridine .
N-(3,5-diaminopyridin-2-yl)-4-méthylpipérazine et leurs sels d'addition

14. Composition selon l'une quelconque des revendications 1 à 13 dans laquelle la ou les bases d'oxydation sont choisies parmi les paraphénylènediamines, les bis-phénylalkylènediamines, les para-aminophénols, les ortho-aminophénols, les bases hétérocycliques et leurs sels d'addition.

15. Composition selon l'une quelconque des revendications 1 à 14 dans laquelle la ou les bases d'oxydation sont chacune présentes en quantité comprise 0,001 et 10 % en poids du poids total de la composition tinctoriale.

16. Composition selon l'une quelconque des revendications 1 à 15, comprenant un ou plusieurs coupleurs additionnels choisis parmi les métaphénylènediamines, les méta-aminophénols, les métadiphénols, les coupleurs naphtaléniques et les coupleurs hétérocycliques autres que les coupleurs de formule (I) et leurs sels d'addition.

17. Composition selon l'une quelconque des revendications 1 à 16 dans laquelle le ou les coupleurs sont présents chacun en quantité comprise entre 0,001 et 10 % en poids du poids total de la composition tinctoriale.

18. Composition selon l'une quelconque des revendications précédentes dans lequel le milieu de teinture est un milieu cosmétique approprié à la teinture des fibres kératiniques.

19. Composition selon l'une quelconque des revendications précédentes contenant de plus un agent oxydant.

20. Procédé de teinture d'oxydation des fibres kératiniques **caractérisé en ce qu'**on applique sur les fibres une composition telle que définie à l'une quelconque des revendications 1 à 18, en présence d'un agent oxydant, pendant un temps suffisant permettant d'obtenir la couleur désirée.

21. Procédé selon la revendication 20, dans lequel l'agent oxydant est choisi parmi le peroxyde d'hydrogène, le peroxyde d'urée, les bromates de métaux alcalins, les persels, les peracides et les enzymes oxydases.

22. Procédé selon l'une des revendications 21 ou 21 dans lequel l'agent oxydant est mélangé au moment de l'emploi à la composition telle que définie selon l'une quelconque des revendications 1 à 18.

23. Procédé selon l'une quelconque des revendications 19 à 21 dans lequel l'agent oxydant est appliqué sur les fibres simultanément ou séquentiellement à la composition telle que définie selon l'une quelconque des revendications 1 à 18 sous forme d'une composition oxydante.

24. Dispositif à plusieurs compartiments dans lequel un premier compartiment tinctoriale contient une composition telle que définie à l'une quelconque des revendications 1 à 18 et un deuxième compartiment contient une composition oxydante.

25. Kit pour la teinture de fibres kératiniques comprenant d'une part une composition telle que définie selon l'une quelconque des revendications 1 à 18 et d'autre part une composition oxydante.

26. Utilisation de la composition telle que définie selon l'une quelconque des revendications 1 à 19 pour la teinture des fibres kératiniques.

27. Composé 2,3,5-triaminopyridine de formule (1) telle que définie dans l'une quelconque des revendications 1 à 13.

28. Composé nitro de formule (II) 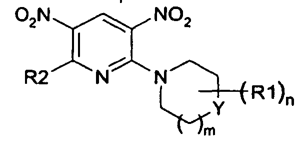 dans laquelle R2, R1, Y, n et m sont tels que définis à l'une quelconque des revendications 1 à 13, à la condition que lorsque R₂ représente l'hydrogène, alors l'héterocycle ne représente pas une pyrrolidine (n = 0, m = 0, Y = CH), ni une pipéridine (n = 0, m = 1, Y = CH), ni une pipérazine (n = 0, m = 1, Y = NH), ni une morpholine (n = 0, m = 1, Y = 0), ni un groupement

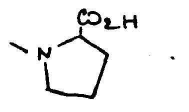

## Claims

1. Dye composition comprising, in a medium that is suitable for dyeing:
- at least one oxidation base, and
- at least one 2,3,5-triaminopyridine coupler of formula (I), or a corresponding addition salt thereof: 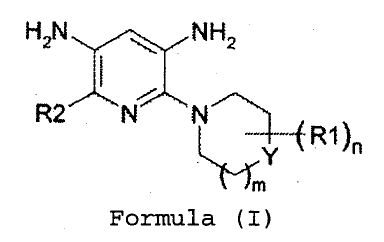
in which:
• R₁ represents:
- a halogen atom;
- a C₁-C₄ alkyl radical optionally substituted with one or more radicals chosen from hydroxyl, carboxyl, (C₁-C₄) alkoxycarbonyl, carboxamido, C₁-C₄ alkylsulfonyl, C₁-C₄ alkoxy, C₁-C₄ alkylsulfonamido NR₃R₄ radicals;
- a carboxyl radical;
- a (C₁-C₄)alkoxycarbonyl radical;
- a carboxamido radical;
- a (C₁-C₄)alkylcarboxamido radical;
- a sulfinic radical;
- a C₁-C₄ alkylsulfonyl radical;
- a C₁-C₄ alkylsulfonamido radical;
- a hydroxyl radical;
- a C₁-C₄ alkoxy radical;
- a C₂-C₄ hydroxyalkoxy radical;
- an amino, monoaminoalkoxy or diaminoalkoxy radical;
- a C₁-C₄ thioether radical;
- a C₁-C₄ alkylsulfoxy radical;
- a sulfonic radical;
- a radical NR₅R₆;
• R₃, R₄, R₅ and R₆, which may be identical or different, represent a hydrogen atom; a C₁-C₄ alkylsulfonyl radical; a (C₁-C₄) alkylcarbonyl radical in which the alkyl radical may be substituted with one or more hydroxyl radicals; an arylcarbonyl radical, the aryl radical possibly being substituted with a radical chosen from hydroxyl, C₁-C₄ alkoxy, amino and (di) (C₁-C₄)alkylamino; a carboxamido radical; a C₁-C₄ alkyl radical optionally substituted with one or more radicals chosen from hydroxyl, C₁-C₄ alkoxy, C₁-C₄ alkylsulfonyl, C₁-C₄ alkylsulfonamido, carboxyl, carboxamido, C₁-C₄ alkylsulfoxy, amino, (di) (C₁-C₄)alkylamino and C₂-C₄ (poly)hydroxyalkylamino radicals;
• R₂ represents a hydrogen atom, a C₁-C₄ alkoxy radical optionally substituted with one or more hydroxyl or C₁-C₂ alkoxy radicals,
• n is an integer between 0 and 7 inclusive,
• m is 0, 1 or 2,
• Y represents an oxygen atom, a radical C(R₈)₂ or a radical NR₇ in which R₇ has the same meaning as R₃ and R₈, which may be identical or different, represent a hydrogen atom or have the same meaning as R₁.

2. Composition according to Claim 1, in which R₁ represents a C₁-C₄ alkoxy radical optionally substituted with one or more hydroxyl, C₁-C₂ alkoxy, amino or (di)alkylamino radicals; a hydroxyl radical; an amino radical; a (di)alkylamino radical; a C₁-C₂ alkyl radical optionally substituted with a hydroxyl or amino.

3. Composition according to Claim 1 or 2, in which R₂ represents a hydrogen atom or an alkoxy radical.

4. Composition according to any one of Claims 1 to 3, in which n represents 0 or 1.

5. Composition according to any one of Claims 1 to 4, in which R₃, R₄, R₅, R₆ and R₇, which may be identical or different, represent a hydrogen atom, a carboxamido radical, a C₁-C₄ alkyl radical optionally substituted with one or more radicals chosen from hydroxyl, C₁-C₄ alkoxy, carboxamido, amino, (di)(C₁-C₄)alkylamino and C₂-C₄ (poly)hydroxyalkylamino radicals.

6. Composition according to Claim 1, in which R₃, R₄, R₅, R₆ and R₇, which may be identical or different, represent a hydrogen atom or a methyl, ethyl, 2-carboxyethyl, 2-hydroxyethyl, 3-hydroxypropyl, 2,3-dihydroxypropyl, 2-hydroxy-3-aminopropyl or 3-hydroxy-2-aminopropyl radical.

7. Composition according to Claim 6, in which R₃, R₄, R₅, R₆ and R₇, which may be identical or different, represent a hydrogen atom, a methyl radical, a 2-hydroxyethyl radical or a 2,3-dihydroxypropyl radical.

8. Composition according to any one of Claims 1 to 7, in which the nitrogen in position 2 of the ring, Y and m form a heterocyclic radical chosen from pyrrolidines, piperidines, homopiperidines, piperazines, homopiperazines and diazepanes.

9. Composition according to Claim 8, in which the heterocycle is chosen from pyrrolidine, 2,5-dimethylpyrrolidine, 2-methylpyrrolidine, proline, 3-hydroxyproline, 4-hydroxyproline, 2,4-dicarboxypyrrolidine, 2-hydroxymethylpyrrolidine, 3-hydroxy-2-hydroxymethylpyrrolidine, 2,5-di(hydroxymethyl)-pyrrolidine, 2-carboxamidopyrrolidine, 3-hydroxy-2-carboxamidopyrrolidine, 2-(dimethylcarboxamido)-pyrrolidine, 2-(dimethylcarboxamido)-3-hydroxypyrrolidine, 3,4-dihydroxy-2-hydroxymethylpyrrolidine, 3-hydroxypyrrolidine, 3,4-dihydroxypyrrolidine, 3-aminopyrrolidine, 3-methylaminopyrrolidine, 3-dimethylaminopyrrolidine, 4-amino-3-hydroxypyrrolidine, 4-methylamino-3-hydroxypyrrolidine, 3-hydroxy-4-(2-hydroxyethyl)aminopyrrolidine, piperidine, 2,6-dimethylpiperidine, 2-carboxypiperidine, 2-carboxamidopiperidine, 2-(dimethylcarboxamido)piperidine, 2-hydroxymethylpiperidine, 3-hydroxy-2-hydroxymethylpiperidine, 3-hydroxypiperidine, 4-hydroxypiperidine, 3-hydroxymethylpiperidine, homopiperidine, 2-carboxyhomopiperidine, 2-carboxamidohomopiperidine, piperazine, 4-methylpiperazine, diazepane, N-methylhomopiperazine and N-β-hydroxyethylhomopiperazine, and the addition salts thereof.

10. Composition according to Claim 8, in which the heterocycle is chosen from pyrrolidine, 2-methylpyrrolidine, 3-hydroxypyrrolidine, 3-aminopyrrolidine, 3-(methylsulfonylamino)pyrrolidine, proline, 3-hydroxyproline, piperidine, hydroxypiperidine, homopiperidine, 4-methylpiperazine, diazepane, N-methylhomopiperazine and N-β-hydroxyethylhomopiperazine, and the addition salts thereof.

11. Composition according to Claim 10, in which the heterocycle is chosen from pyrrolidine, 3-hydroxypyrrolidine, 3-aminopyrrolidine, 3-(methylsulfonylamino)pyrrolidine, proline and 3-hydroxyproline.

12. Composition according to Claim 1, in which R₁ represents an alkyl, amino, hydroxyalkyl or hydroxyl radical, R₂ is hydrogen, m is 0 or 1 and n is between 0 and 2.

13. Composition according to any one of Claims 1 to 12, in which the compounds of formula (I) are chosen from:
N-(3,5-diaminopyrid-2-yl)pyrrolidine
N-(3,5-diaminopyrid-2-yl)-2-methylpyrrolidine
N-(3,5-diaminopyrid-2-yl)-2,5-dimethylpyrrolidine
N-(3,5-diaminopyrid-2-yl)-2-hydroxymethylpyrrolidine
N-(3,5-diaminopyrid-2-yl)proline
N-(3,5₋diaminopyrid-2-yl)-3-hydroxypyrrolidine
N-(3,5-diaminopyrid-2-yl)-2-carboxamidopyrrolidine
N-(3,5-diaminopyrid-2-yl)-2-dimethylcarboxamidopyrrolidine
N-(3,5-diaminopyrid-2-yl)-3,4-dihydroxypyrrolidine
N-(3,5-diaminopyrid-2-yl)-3-aminopyrrolidine
N-(3,5-diaminopyrid-2-yl)-3-dimethylaminopyrrolidine
N-(3,5-diaminopyrid-2-yl)piperidine
N-(3,5-diaminopyrid-2-yl)-4-methylpiperazine and the addition salts thereof.

14. Composition according to any one of Claims 1 to 13, in which the oxidation base(s) is (are) chosen from para-phenylenediamines, bis(phenyl)alkylenediamines, para-aminophenols, ortho-aminophenols and heterocyclic bases, and the addition salts thereof.

15. Composition according to any one of Claims 1 to 14, in which the oxidation base(s) is (are) each present in an amount of between 0.001% and 10% by weight relative to the total weight of the dye composition.

16. Composition according to any one of Claims 1 to 15, comprising one or more additional couplers chosen from meta-phenylenediamines, meta-aminophenols, metadiphenols, naphthalene-based couplers and heterocyclic couplers other than the couplers of formula (I), and the addition salts thereof.

17. Composition according to any one of Claims 1 to 16, in which the coupler(s) is (are) each present in an amount of between 0.001% and 10% by weight relative to the total weight of the dye composition.

18. Composition according to any one of the preceding claims, in which the dyeing medium is a cosmetic medium that is suitable for dyeing keratin fibres.

19. Composition according to any one of the preceding claims, also comprising an oxidizing agent.

20. Process for the oxidation dyeing of keratin fibres, **characterized in that** a composition as defined in any one of Claims 1 to 18 is applied to the fibres, in the presence of an oxidizing agent, for a time that is sufficient to allow the desired colour to be obtained.

21. Process according to Claim 20, in which the oxidizing agent is chosen from hydrogen peroxide, urea peroxide, alkali metal bromates, persalts, peracids and oxidase enzymes.

22. Process according to either of Claims 20 and 21, in which the oxidizing agent is mixed at the time of use with the composition as defined according to any one of Claims 1 to 18.

23. Process according to any one of Claims 19 to 21, in which the oxidizing agent is applied to the fibres simultaneously with or sequentially to the composition as defined in any one of Claims 1 to 18, in the form of an oxidizing composition.

24. Multi-compartment device, in which a first dye compartment contains a composition as defined in any one of Claims 1 to 18 and a second compartment contains an oxidizing composition.

25. Kit for dyeing keratin fibres, on the one hand containing a composition as defined according to any one of Claims 1 to 18, and on the other hand containing an oxidizing composition.

26. Use of the composition as defined according to any one of Claims 1 to 19 for dyeing keratin fibres.

27. 2,3,5-Triaminopyridine compound of formula (I) as defined in any one of Claims 1 to 13.

28. Nitro compound of formula (II) 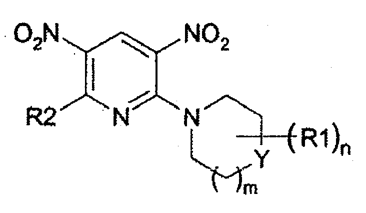 in which R₂, R₁, Y, n and m are as defined in any one of Claims 1 to 13, with the condition that when R₂ represents hydrogen, then the heterocycle does not represent a pyrrolidine (n = 0, m = 0, Y = CH), a piperidine (n = 0, m = 1, Y = CH), a piperazine (n = 0, m = 1, Y = NH), a morpholine (n = 0, m = 1, Y = 0) or a group

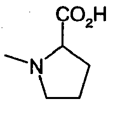

## Patentansprüche

1. Farbmittelzusammensetzung, die in einem zum Färben geeigneten Medium enthält:
- mindestens eine Oxidationsbase, und
- mindestens einen 2,3,5-Triaminopyridinkuppler der Formel (I) oder eines seiner entsprechenden Additionssalze: 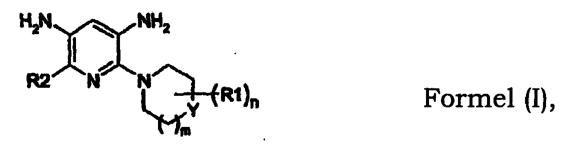
wobei in der Formel:
• R₁ bedeutet:
- ein Halogenatom;
- eine C₁₋₄-Alkylgruppe, die gegebenenfalls mit einer oder mehreren Gruppen substituiert ist, die unter den Gruppen Hydroxy, Carboxy, Alkoxycarbonyl mit 1 bis 4 Kohlenstoffatomen, Carboxamido, Alkyl(C₁₋₄)sulfonyl, C₁₋₄-Alkoxy, Alkyl(C₁₋₄)sulfonamido, NR₃R₄ ausgewählt ist;
- eine Carboxygruppe;
- eine C₁₋₄-Alkoxycarbonylgruppe;
- eine Carboxamidogruppe;
- eine C₁₋₄-Alkylcarboxamidogruppe;
- eine Sulfinsäuregruppe;
- eine Alkyl(C₁₋₄)sulfonylgruppe;
- eine Alkyl(C₁₋₄)sulfonamidogruppe;
- eine Hydroxygruppe;
- eine C₁₋₄-Alkoxygruppe;
- eine C₂₋₄-Hydroxyalkoxygruppe;
- eine Aminogruppe oder Mono- oder Diaminoalkoxygruppe;
- eine C₁₋₄-Thioethergruppe;
- eine Alkyl(C₁₋₄)sulfoxidgruppe;
- eine Sulfonsäuregruppe;
- eine Gruppe NR₅R₆;
• R₃, R₄, R₅ und R₆, die gleich oder verschieden sind, bedeuten: ein Wasserstoffatom; eine Alkyl(C₁₋₄)sulfonylgruppe; eine Alkyl(C₁₋₄)carbonylgruppe, bei der die Alkylgruppe mit einer oder mehreren Hydroxygruppen substituiert sein kann; eine Arylcarbonylgruppe, eine Arylgruppe, die mit einer Gruppe substituiert sein kann, die unter Hydroxy, C₁₋₄-Alkoxy, Amino oder (Di)alkyl(C₁₋₄)amino ausgewählt sein kann; eine Carboxamidogruppe; eine C₁₋₄-Alkylgruppe, die gegebenenfalls mit einer oder mehreren Gruppen substituiert ist, die unter den Gruppen Hydroxy, C₁₋₄-Alkoxy, Alkyl(C₁₋₄)sulfonyl, Alkyl(C₁₋₄)sulfonamido, Carboxy, Carboxamido, Alkyl(C₁₋₄)sulfoxid, Amino, (Di)(C₁₋₄)alkylamino oder (Poly)hydroxyalkylamino mit 2 bis 4 Kohlenstoffatomen ausgewählt sind;
• R₂ bedeutet: ein Wasserstoffatom; eine C₁₋₄-Alkoxygruppe, die gegebenenfalls mit einer oder mehreren Gruppen Hydroxy, C₁₋₂-Alkoxy substituiert ist;
• n 0 oder eine ganze Zahl von 1 bis 7, wobei die Grenzen eingeschlossen sind,
• m 0, 1 oder 2,
• Y ein Sauerstoffatom, eine Gruppe C(R₈)₂ oder eine Gruppe NR₇, wobei R₇ die gleiche Bedeutung hat wie R₃ und die Gruppen R₈, die gleich oder verschieden sind, ein Wasserstoffatom bedeuten oder die gleiche Bedeutung haben wie R₁.

2. Zusammensetzung nach Anspruch 1, wobei R₁ eine C₁₋₄-Alkoxygruppe bedeutet, die gegebenenfalls mit einer oder mehreren Hydroxygruppen, C₁₋₂-Alkoxygruppen, Aminogruppen oder (Di)alkylaminogruppen substituiert ist; eine Hydroxygruppe; eine Aminogruppe; eine (Di)alkylaminogruppe; eine C₁₋₂-Alkylgruppe, die gegebenenfalls mit Hydroxy, Amino substituiert ist.

3. Zusammensetzung nach Anspruch 1 oder 2, wobei R₂ ein Wasserstoffatom oder eine Alkoxygruppe bedeutet.

4. Zusammensetzung nach einem der Ansprüche 1 bis 4, wobei n 0 oder 1 bedeutet.

5. Zusammensetzung nach einem der Ansprüche 1 bis 4, wobei die Gruppen R₃, R₄, R₅, R₆ und R₇, die gleich oder verschieden sind, ein Wasserstoffatom, eine Carboxamidogruppe, eine C₁₋₄-Alkylgruppe, die gegebenenfalls mit einer oder mehreren Gruppen substituiert ist, die unter den Gruppen Hydroxy, C₁₋₄-Alkoxy, Carboxamido, Amino, (Di)(C₁₋₄)alkylamino, (Poly)hydroxyalkylamino mit 2 bis 4 Kohlenstoffatomen ausgewählt sind.

6. Zusammensetzung nach Anspruch 1, wobei die Gruppen R₃, R₄, R₅, R₆ und R₇, die gleich oder verschieden sind, ein Wasserstoffatom, Methyl, Ethyl, 2-Carboxyethyl, 2-Hydroxyethyl, 3-Hydroxypropyl, 2,3-Dihydroxypropyl, 2-Hydroxy-3-aminopropyl, 3-Hydroxy-2-aminopropyl bedeuten.

7. Zusammensetzung nach Anspruch 6, wobei die Gruppen R₃, R₄, R₅, R₆ und R₇, die gleich oder verschieden sind, ein Wasserstoffatom, Methyl, 2-Hydroxyethyl, 2,3-Dihydroxypropyl bedeuten.

8. Zusammensetzung nach einem der Ansprüche 1 bis 7, wobei der Stickstoff in 2-Stellung des Rings, Y und m eine heterocyclische Gruppe bilden, die unter den Pyrrolidinen, Piperidinen, Homopiperidinen, Piperazinen, Homopiperazinen, Diazepanen ausgewählt ist.

9. Zusammensetzung nach Anspruch 8, wobei der Heterocyclus ausgewählt ist unter Pyrrolidin, 2,5-Dimethylpyrrolidin, 2-Methylpyrrolidin, Prolin, 3-Hydroxyprolin, 4-Hydroxyprolin, 2,4-Dicarboxypyrrolidin, 2-Hydroxymethylpyrrolidin, 3-Hydroxy-2-hydroxymethylpyrrolidin, 2,5-Di(hydroxymethyl)pyrrolidin, 2-Carboxamidopyrrolidin, 3-Hydroxy-2-carboxamidopyrrolidin, 2-(Dimethylcarboxamido)-pyrrolidin, 2-(Dimethylcarboxamido)-3-hydroxypyrrolidin, 3,4-Dihydroxy-2-hydroxymethylpyrrolidin, 3-Hydroxypyrrolidin, 3,4-Dihydroxypyrrolidin, 3-Aminopyrrolidin, 3-Methylaminopyrrolidin, 3-Dimethylaminopyrrolidin, 4-Amino-3-hydroxypyrrolidin, 4-Methylamino-3-hydroxypyrrolidin, 3-Hydroxy-4-(2-hydroxyethyl)-aminopyrrolidin, Piperidin, 2,6-Dimethylpiperidin, 2-Carboxypiperidin, 2-Carboxamidopiperidin, 2-(Dimethylcarboxamido)-piperidin, 2-Hydroxymethylpiperidin, 3-Hydroxy-2-hydroxymethylpiperidin, 3-Hydroxypiperidin, 4-Hydroxypiperidin, 3-Hydroxymethylpiperidin, Homopiperidin, 2-Carboxyhomopiperidin, 2-Carboxamidohomopiperidin, Piperazin, 4-Methylpiperazin, Diazepan, N-Methylhomopiperazin, N-β-Hydroxyethylhomopiperazin und deren Additionssalzen mit einer Säure.

10. Zusammensetzung nach Anspruch 8, wobei der Heterocyclus unter Pyrrolidin, 2-Methylpyrrolidin, 3-Hydroxypyrrolidin, 3-Aminopyrrolidin, 3-(Methylsulfonylamino)-pyrrolidin, Prolin, 3-Hydroxyprolin, Piperidin, Hydroxypiperidin, Homopiperidin, 4-Methylpiperazin, Diazepan, N-Methylhomopiperazin, N-β-Hydroxyethylhomopiperazin und deren Additionssalzen mit einer Säure ausgewählt ist.

11. Zusammensetzung nach Anspruch 10, wobei der Heterocyclus unter Pyrrolidin, 3-Hydroxypyrrolidin, 3-Aminopyrrolidin, 3-(Methylsulfonylamino)-pyrrolidin, Prolin, 3-Hydroxyprolin ausgewählt ist.

12. Zusammensetzung nach Anspruch 1, wobei R₁ eine Gruppe Alkyl, Amino, Hydroxyalkyl, Hydroxy bedeutet, R₂ Wasserstoff ist, m 0 oder 1 bedeutet und n 0, 1 oder 2 bedeutet.

13. Zusammensetzung nach einem der Ansprüche 1 bis 12, wobei die Verbindungen der Formel (I) ausgewählt sind unter:
N-(3,5-Diaminopyridin-2-yl)-pyrrolidin,
N-(3, 5-Diaminopyridin-2-yl)-2-methylpyrrolidin,
N-(3,5-Diaminopyridin-2-yl)-2,5-dimethylpyrrolidin,
N-(3,5-Diaminopyridin-2-yl)-2-hydroxymethylpyrrolidin,
N-(3, 5-Diaminopyridin-2-yl)-prolin,
N-(3,5-Diaminopyridin-2-yl)-3-hydroxypyrrolidin,
N-(3, 5-Diaminopyridin-2-yl)-2-carboxamidopyrrolidin,
N-(3,5-Diaminopyridin-2-yl)-2-dimethylcarboxamidopyrrolidin,
N-(3, 5-Diaminopyridin-2-yl)-3,4-dihydroxypyrrolidin,
N-(3, 5-Diaminopyridin-2-yl)-3-aminopyrrolidin,
N-(3,5-Diaminopyridin-2-yl)-3-dimethylaminopyrrolidin,
N-(3,5-Diaminopyridin-2-yl)-piperidin,
N-(3,5-Diaminopyridin-2-yl)-4-methylpiperazin und deren Additionssalzen.

14. Zusammensetzung nach einem der Ansprüche 1 bis 13, bei der die Oxidationsbase(n) unter den p-Phenylendiaminen, Bisphenylalkylendiaminen, p-Aminophenolen, o-Aminophenolen, heterocyclischen Basen und deren Additionssalzen ausgewählt sind.

15. Zusammensetzung nach einem der Ansprüche 1 bis 14, bei der die Oxidationsbase(n) jede in einer Menge von 0,001 bis 10 Gew.-% des Gesamtgewichts der Farbmittelzusammensetzung enthalten sind.

16. Zusammensetzung nach einem der Ansprüche 1 bis 15, die einen oder mehrere ergänzende Kuppler enthält, die unter den m-Phenylendiaminen, m-Aminophenolen, m-Dihydroxybenzolen, Naphthalinkupplern und heterocyclischen Kupplern, die von den Kupplern der Formel (I) verschieden sind, und deren Additionssalzen ausgewählt sind.

17. Zusammensetzung nach einem der Ansprüche 1 bis 16, wobei der oder die Kuppler jeder in einer Menge von 0,001 bis 10 Gew.-% des Gesamtgewichts der Farbmittelzusammensetzung enthalten sind.

18. Zusammensetzung nach einem der vorhergehenden Ansprüche, wobei das Medium zum Färben ein zum Färben von Keratinfasern geeignetes kosmetisches Medium ist.

19. Zusammensetzung nach einem der vorhergehenden Ansprüche, die ferner ein Oxidationsmittel enthält.

20. Verfahren zum oxidativen Färben von Keratinfasern, **dadurch gekennzeichnet, dass** auf die Fasern eine Zusammensetzung nach einem der Ansprüche 1 bis 18 in Gegenwart eines Oxidationsmittels während einer Zeitspanne aufgebracht wird, die ausreichend ist, um die gewünschte Farbe zu bilden.

21. Verfahren nach Anspruch 20, wobei das Oxidationsmittel unter Wasserstoffperoxid, Harnstoffperoxid, Alkalimetallbromaten, Salzen von Persäuren, Persäuren und Oxidasen ausgewählt ist.

22. Verfahren nach einem der Ansprüche 20 oder 21, wobei das Oxidationsmittel bei einer Anwendung mit der in einem der Ansprüche 1 bis 18 definierten Zusammensetzung vermischt wird.

23. Verfahren nach einem der Ansprüche 19 bis 21, wobei das Oxidationsmittel auf die Fasern gleichzeitig mit oder nach der Zusammensetzung, die in einem der Ansprüche 1 bis 18 definiert ist, in Form einer oxidierenden Zusammensetzung aufgebracht wird.

24. Vorrichtung mit mehreren Abteilungen, wobei eine Abteilung zum Färben eine Zusammensetzung enthält, wie sie in einem der Ansprüche 1 bis 18 definiert ist, und eine zweite Abteilung eine oxidierende Zusammensetzung enthält.

25. Kit zum Färben von Keratinfasern, das einerseits eine Zusammensetzung nach einem der Ansprüche 1 bis 18 und andererseits eine oxidierende Zusammensetzung umfasst.

26. Verwendung der Zusammensetzung nach einem der Ansprüche 1 bis 19 zum Färben von Keratinfasern.

27. 2,3,5-Triaminopyridin der Formel (I) nach einem der Ansprüche 1 bis 13.

28. Nitroverbindung der Formel (II) 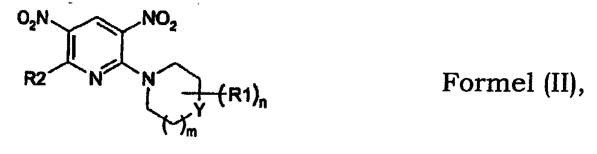 wobei R2, R1, Y, n und m die in einem der Ansprüche 1 bis 13 angegebenen Bedeutungen aufweisen, mit der Maßgabe, dass der Heterocyclus weder ein Pyrrolidin (n = 0, m = 0, Y = CH) noch ein Piperidin (n = 0, m = 1, Y = CH), Piperazin (n = 0, m = 1, Y = NH), Morpholin (n = 0, m = 1, Y = O) oder eine Gruppe 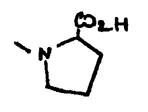 bedeutet.
